# EUROPÄISCHE PATENTANMELDUNG

(11) **EP 3 586 915 A1**
(43) Veröffentlichungstag der Anmeldung: **01.01.2020**
(21) Anmeldenummer: 18202080.0
(22) Anmeldetag: 23.10.2018
(51) Int. Cl.: A61N 5/06, A61N 1/372, A61N 1/36, A61N 1/368, A61N 1/362, A61B 5/00, A61B 5/046, A61B 5/0464, A61N 5/10

(54) **VORRICHTUNG ZUR AKTIVIERUNG VON ZELLSTRUKTUREN MITTELS ELEKTROMAGNETISCHER ENERGIE**

(30) Priorität: 25.06.2018 DE 102018115180
(71) Anmelder: BIOTRONIK SE & Co. KG, 12359 Berlin (DE)
(72) Erfinder: Dörr, Thomas, 12437 Berlin (DE); Weiss, Ingo, 12435 Berlin (DE)
(74) Vertreter: Keck, Hans-Georg

(57) **Zusammenfassung**

Es wird eine Vorrichtung vorgeschlagen, mindestens umfassend:
- eine Energiequelle,
- einen Energiespeicher,
- eine Elektronikeinheit,
wobei die Vorrichtung zur Implantation im menschlichen oder tierischen Körper ausgelegt ist,
- einen Aktuator, der mit dem Energiespeicher gekoppelt ist und dazu ausgelegt ist, durch Entladung des Energiespeichers elektromagnetische Wellen auszusenden.

## Beschreibung

Die Erfindung beschreibt eine Vorrichtung und Methode zur Aktivierung von Zellstrukturen mittels elektromagnetischer Energie.

Seit langem bekannt sind Stimulatoren zur Anregung von menschlichem oder tierischem Gewebe durch elektrischen Strom. Elektrische Stimulation von Nervengewebe bewirkt die Aktivierung von Aktionspotentialen, die in der medizinischen Therapie und Diagnostik genutzt wird. Beispiele für implantierbare Stimulatoren sind u.a. implantierbare Herzschrittmacher und -Defibrillatoren, Rückenmarkstimulatoren, Vagusnervstimulatoren, Hirnschrittmacher.

Elektrische Stimulationsvorrichtungen sind galvanisch an menschliches oder tierisches Gewebe gekoppelt und messen elektrische Potentiale und/oder stimulieren das Gewebe durch elektrische Stromabgabe zur Auslösung von Aktionspotentialen. Nachteile der elektrischen Stimulation ergeben sich unter anderem durch die notwendige galvanische Verbindung zum Gewebe. Die direkte Kontaktierung Stimulationselektroden mit dem Gewebe und Einkopplung von Strom können z.B. Nekrosen, elektrische Nachpotentiale, die die Sensingfunktionen limitieren, Crosstalk (d.h. Interferenzen bei paralleler Stimulation über verschiedene Stimulationspfade), ungewollte externe Induktion von Therapieströmen (z.B. bewirkt durch die hochfrequenten Magnetfelder innerhalb eines Magnetresonanztomografen), eventuelle Schmerzereignisse, Elektroporation oder ungewollte Stimulation in der Nähe befindlichen Gewebes (z.B. kann bei Herzstimulatoren der Nervus Phrenicus ungewollt mitstimuliert werden) verursachen. Weitere Nachteile ergeben sich durch die mit dem Therapiestrom verbundenen Einrichtungen zur Stromgenerierung, die aufwändig und voluminös sein können (z.B. Ladeschaltung bei einem implantierbaren Kardioverter-Defibrillator) und Limitationen in der Erreichbarkeit des Zielgewebes aufweisen.

Seit einigen Jahren ist die genetische Manipulation von Gewebe zur Generierung einer Erregbarkeit mittels elektromagnetischer Wellen Gegenstand der wissenschaftlichen Diskussion und Forschung. Dabei ist es gelungen, Zielgewebe genetisch so zu verändern, dass mittels elektromagnetischen Wellen Aktionspotentiale evoziert werden können.

Eine dieser Methoden wird als optogenetische Manipulation bezeichnet. Diese wird beispielsweise beschrieben in
- Lung MS, Pilowsky P, Goldys EM., "Activation of the Mammalian cells by using light-sensitive ion channels." Methods Mol Biol. 2012;875:241-51; die Publikation beschreibt optogenetische Gewebemanipulation zum Zwecke der Auslösung von Aktionspotentialen mittels Licht im sichtbaren Spektrum;
- Wang et al. "Optogenetic Control of Heart Rhythm by Selective Stimulation of Cardiomyocytes Derived from Pnmt+ Cells in Murine Heart", Januar 2017 in Scientific Reports.

US 2009/0088680 A1 beschreibt ein katheterbasiertes System zur Einbringung eines Lichtleiters in das Herz, wobei das Herzgewebe zuvor mittels optogenetischer Manipulation behandelt wurde. Um den Lichtleiter in das Herzinnere zu befördern, wird ein Gefäßzugang beim Patienten gelegt, ein Katheter wird in das Herz vorgeschoben und der Lichtleiter durch den Katheter in das Herzinnere gebracht. Über den Lichtleiter wird Licht in das Herz geleitet und eine endokardiale Stimulation des manipulierten Gewebes durchgeführt.

Ein Nachteil der in US 2009/0088680 A1 beschriebenen Methode besteht darin, dass sie durch einen Arzt ambulant durchgeführt werden und der Lichtleiter muss von einem externen Gerät betrieben werden muss. Nachteile entstehen auch durch den venösen Gefäßzugang und die Katheterisierung wie zum Beispiel Infektionen und Gefäßverschlüsse.

Die Aufgabe der Erfindung ist es, in einer dauerhaft implantierbaren Vorrichtung Aktionspotentiale an vorbehandelten tierischen und/oder menschlichen erregbaren Zellstrukturen zu evozieren, modulieren, terminieren und/oder zu inhibieren ohne dass es einer galvanischen Einprägung elektrischem Stromes bedarf.

Die Aufgabe ist es ferner, Stimulationstherapie mit elektromagnetischer Strahlung von manipuliertem Gewebe mittels gebräuchlicher implantierbarer Pulsgeneratoren (IPGs)s zu ermöglichen.

Eine Aufgabe der vorliegenden Erfindung ist die Bereitstellung einer Vorrichtung und eines Systems, das die genannten Nachteile nicht aufweist.

Eine weitere Aufgabe der vorliegenden Erfindung besteht in der Bereitstellung einer Vorrichtung und eines Systems für die Stimulation von Gewebe mittels elektromagnetischen Wellen zur Auslösung von Aktionspotentialen, wobei die Vorrichtung eine dauerhafte Stimulation ermöglicht, ohne dass ein permanenter Gefäßzugang, externe Geräte oder eine ambulante Sitzung beim Arzt erforderlich ist.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Gemäß einem Aspekt der vorliegenden Erfindung wird eine Stimulationsvorrichtung vorgeschlagen, die zumindest umfasst:
eine Energiequelle,
eine Elektronikeinheit,
einen Aktuator, der mit der Elektronik und/oder der Energiequelle gekoppelt ist, und wobei der Aktuator ausgelegt ist, elektromagnetische Wellen zur Stimulation von genetisch manipuliertem Gewebe auszusenden. Die Stimulationsvorrichtung ist zur mindestens temporären Implantation im menschlichen oder tierischen Körper ausgelegt. Die Elektronikeinheit umfasst eine Steuereinheit, die dazu ausgelegt ist, besagtes Gewebe mittels der elektromagnetischen Wellen des Aktuators zu stimulieren.

Gemäß eines Ausführungsbeispiels der Erfindung ist der Aktuator dazu ausgelegt, elektromagnetische Wellen zur Stimulation von optogenetisch manipuliertem Gewebe auszusenden.

Mit einer implantierbaren Vorrichtung zur Stimulation von genetisch manipuliertem Gewebe werden die zuvor adressierten Probleme gelöst. Durch ein Implantat kann eine dauerhafte Stimulation bereitgestellt werden, ohne dass eine Katheterisierung des Patienten oder ein Eingriff durch den Arzt erforderlich wären.

Vorzugsweise ist die Stimulation durch die erfindungsgemäße Stimulationsvorrichtung dazu ausgelegt, mindestens eine der folgenden Erkrankungen zu therapieren:
- kardiale Bradykardie,
- kardiale Tachykardie.
- atriale Fibrillation,
- ventrikuläre Fibrillation,
- Herzinsuffizienz,
- Parkinsonkrankheit,
- Tremor,
- Dystonie,
- Depression,
- Tourette-Syndrom,
- chronische Schmerzen,
- Epilepsie,
- neuronale oder muskuläre Störungen.

Gemäß eines Ausführungsbeispiels der erfindungsgemäßen Stimulationsvorrichtung ist der Aktuator dazu ausgelegt, elektromagnetische Wellen im Frequenzspektrum von infrarotem Licht bis Röntgenstrahlung auszusenden. Gemäß einer bevorzugten Ausführungsform ist der Aktuator dazu ausgelegt, elektromagnetische Wellen im Frequenzspektrum zwischen 10¹³ und 10²⁰ Hz auszusenden. Bevorzugt ist der Aktuator dazu ausgelegt, elektromagnetische Wellen im Frequenzspektrum zwischen 10¹³ und 10¹⁶ Hz auszusenden, was einem Spektrum zwischen einschließlich Infrarotstrahlung bis zur Ultraviolettstrahlung entspricht. Gemäß eines Ausführungsbeispiels ist der Aktuator ausgelegt, elektromagnetische Wellen im Frequenzspektrum zwischen 10¹⁴ und 10¹⁵ Hz auszusenden, was in etwa dem für das menschliche Auge sichtbare Spektrum entspricht. Ein Vorteil dieser Wahl ist die verhältnismäßig einfache Implementierung eines solchen Aktuators, sowie geringer Energiebedarf und die Unschädlichkeit des Lichts für menschliches oder tierisches Gewebe. Im Rahmen der vorliegenden Erfindung sind die Angaben der Frequenzbereichsgrenzen nicht als harte Grenze zu interpretieren, sondern die Frequenzbereichsgrenze soll annähernd in der angegebenen Frequenz mit gewisser Toleranz liegen. Die Toleranz soll sich anhand des Verständnisses des Fachmanns orientieren. Insbesondere soll sich die Toleranz daran orientieren, welche Frequenzen der Fachmann im elektromagnetischen Spektrum einem entsprechenden Strahlungstyp (z.B. Spektrum des sichtbaren Lichts, Röntgenstrahlung, Infrarotstrahlung) zuordnet.

In einer bevorzugten Ausgestaltung der Erfindung umfasst die Stimulationsvorrichtung mindestens ein Gehäuse, wobei das Gehäuse biokompatibles Material umfasst. Bevorzugterweise besteht der Teil der Stimulationsvorrichtung, das im implantierten Zustand in direktem Kontakt mit menschlichem oder tierischem Gewebe steht, aus biokompatiblem Material, sodass die Stimulationsvorrichtung durch den Organismus nicht als Fremdkörper erkannt wird und somit eine biologische Abwehrreaktion verhindert wird.

Gemäß einer Ausführungsform der vorliegenden Erfindung ist das Gehäuse hermetisch verschlossen, sodass im implantierten Zustand keine Körperflüssigkeiten ins Innere des Gehäuses eindringen können.

Nach einem weiteren Aspekt der erfindungsgemäßen Stimulationsvorrichtung ist die Elektronikeinheit innerhalb des Gehäuses angeordnet, wobei die Energiequelle und/oder der Aktuator innerhalb des Gehäuses oder außerhalb des Gehäuses angeordnet sind/ist. Die Anordnung von Komponenten innerhalb eines Gehäuses bietet den Vorteil, dass die Kompaktheit der Vorrichtung erhöht und Verdrahtungswege minimiert werden. Auch sind alle Komponenten innerhalb des Gehäuses vor äußeren Einflüssen geschützt. Bei der Anordnung des Aktuators außerhalb des Gehäuses bietet sich der Vorteil, dass dadurch größere Flexibilität bei der Platzierung des Aktuators ermöglicht wird. Auf diese Weise kann der Aktuator so platziert werden, dass das zu stimulierende Gebiet örtlich gezielter mit elektromagnetischen Wellen bestrahlt wird. Werden eine aufladbare Batterie und ein Gehäuse aus elektrisch leitendem Material verwendet, so bietet eine Anordnung der Batterie außerhalb des Gehäuses den Vorteil, dass ein Aufladeprozess (z.B. induktiv durch eine externe Spule) effizienter erfolgt als bei einer innerhalb des Gehäuses angeordneten Batterie, da Wirbelstromverluste durch das Gehäusematerial verringert werden.

Des Weiteren, gemäß einer Ausführungsform der erfindungsgemäßen Stimulationsvorrichtung, weist die Elektronikeinheit mindestens eine der folgenden oder eine Kombination aus den folgenden Einheiten auf:
- eine Aufhahmeeinheit ausgelegt zur Messung von Daten, die die Gewebeaktivität und/oder Erfolg einer Stimulation charakterisieren, und/oder
- eine Auswerteeinheit zur Auswertung von Messdaten hinsichtlich des Erfordernisses einer Stimulation und/oder hinsichtlich des Erfolgs einer Stimulation.

Zum Beispiel kann die Steuereinheit dazu ausgelegt sein, für die Stimulation eine der folgenden Eigenschaften oder eine Kombination der folgenden Eigenschaften der elektromagnetischen Wellen zu variieren:
- die Intensität, d.h. die Amplitude der elektromagnetischen Wellen,
- die Frequenz,
- die Dauer eines Wellenzugs, und/oder
- das Tastverhältnis der elektromagnetischen Wellen, d.h. Verhältnis zwischen Aussenden einer elektromagnetischen Welle und Pausieren über die Zeit, um somit eine Modulation der elektromagnetischen Welle bzw. dem elektromagnetischen Signal durchzuführen.

Gemäß eines Aspekts der vorliegenden Erfindung beträgt die Dauer eines Wellenzugs 0,1ms-5s in Abhängigkeit der beabsichtigten Wirkung der Stimulation durch die elektromagnetischen Wellen. Dabei wird unter einem Wellenzug eine durchgehende elektromagnetische Welle verstanden. Beispielsweise kann ein Wellenzug mit einer Dauer von 0,1ms-2ms gewählt werden für eine Anwendung in der Neurostimulation, für die Stimulation des Herzens als Schrittmacherstimulus oder für das Antitachycardia Pacing (ATP) als Dauer für einen Stimulus in einer Sequenz von ATP-Stimuli. Eine Dauer von 0.1s-5s kann z.B. gewählt werden für die Stimulation des Herzens als Kardioversions- oder Defibrillations-Stimulus.

Des Weiteren, gemäß eines Ausführungsbeispiels der vorliegenden Erfindung, liegt die Frequenz der Stimulation mittels elektromagnetischer Wellen im Frequenzspektrum zwischen 10¹³ und 10²⁰ Hz. Gemäß eines bevorzugten Ausführungsbeispiels wird eine Frequenz im Bereich: 10¹³-10¹⁶ oder 10¹⁴ - 10¹⁵ Hz gewählt.

Nach einem weiteren Aspekt der erfindungsgemäßen Stimulationsvorrichtung weist die Stimulationsvorrichtung mindestens eine Elektrodenleitung oder Elektrodensonde auf. Zum Beispiel kann die Stimulationsvorrichtung eine langgestreckte, flexible Elektrodenleitung aufweisen, wie sie für gebräuchliche Herzschrittmacher oder Neurostimulatoren eingesetzt werden. Gemäß einer Ausführungsform kann der Aktuator kann am distalen Ende einer solchen Elektrodenleitung angeordnet sein.

Zum Beispiel, gemäß einer Ausführungsform, kann die Aufnahmeeinheit dazu ausgelegt sein, elektromagnetische Wellen zu empfangen und in elektrisch speicherbare Messdaten umzuwandeln. Eine solche Aufnahmeeinheit kann einen Lichtsensor umfassen, wie beispielsweise einen Fotodetektor. Die aufgenommene Information der elektromagnetischen Wellen wird in Messdaten umgewandelt und kann dann mittels der Auswerteeinheit analysiert werden. Die Auswerteeinheit kann die Messdaten beispielsweise hinsichtlich verschiedener Parameter auswerten, die einen physiologischen Zustand des Patienten und/oder einen Zustand der Stimulationsvorrichtung selbst repräsentieren. Beispielhafte Parameter wären: Erfolg einer Stimulationstherapie, Bedarfszustand einer Stimulationstherapie, ein gesundheitlicher Zustand des Patienten, einen Zustandsparameter der Umgebung der Stimulationsvorrichtung, etc.

Des Weiteren, gemäß einer bevorzugten Ausführungsform der vorliegenden Erfindung, sind mindestens die Energiequelle und die Elektronikeinheit innerhalb des Gehäuses angeordnet. Diese Einheiten sind empfindlich gegenüber den zum Teil aggressiven Körperflüssigkeiten, denen sie im implantierten Zustand ausgesetzt wären. Daher ist gemäß einer Ausführungsform die Platzierung in einem hermetisch abgeschlossenen Gehäuse vorteilhaft.

In einer bevorzugten Ausführungsform ist die Elektronikeinheit mindestens teilweise durch ein externes Gerät konfigurierbar. Die Konfiguration kann drahtlos oder, durch Ankopplung eines externen Geräts an die Elektronikeinheit, drahtgebunden stattfinden. Auf diese Weise sind Funktionen der erfindungsgemäßen Stimulationsvorrichtung flexibel programmierbar und auf einen Patienten individuell anpassbar.

Gemäß eines weiteren Aspekts der vorliegenden Erfindung umfasst die Stimulationsvorrichtung mindestens eine Fixiereinheit, die dazu ausgelegt ist, zumindest einen Teil der Stimulationsvorrichtung in Gewebe eines menschlichen oder tierischen Körpers zu fixieren. Beispiele einer solchen Fixiereinheit sind: Schraubelemente, Hakenelemente, Ankerelemente, Gewebekleber.

Gemäß einer beispielhaften Ausführung der vorliegenden Erfindung umfasst die Energiequelle eine Batterie, die als Primärzelle oder Sekundärzelle gestaltet ist. Die Batterie ist vorzugsweise im Inneren des Gehäuses angeordnet. Gemäß eines Ausführungsbeispiels ist die Batterie wiederaufladbar, z.B. induktiv über ein externes Aufladegerät mit einer Aufladespule.

Des Weiteren, gemäß einer bevorzugten Ausführungsform der vorliegenden Erfindung, weist die Stimulationsvorrichtung eine Telemetrieeinheit zur Drahtloskommunikation mit mindestens einem externen Gerät und/oder Datenzentrum auf. So können Messdaten durch die Stimulationsvorrichtung versendet und andere Daten empfangen werden, wie zum Beispiel Programmieraufträge, patientenspezifische Daten etc.

Gemäß einer Ausführungsform der vorliegenden Erfindung umfasst der Aktuator mindestens einen Lichtwellenleiter. Der Lichtwellenleiter dient zur Einkopplung der elektromagnetischen Wellen zur Stimulation von optogenetischem Gewebe.

Des Weiteren kann, gemäß weiteren Ausführungsformen der vorliegenden Erfindung, die Stimulationsvorrichtung einen der folgenden Sensoren oder eine Kombination der folgenden Sensoren umfassen:
- ein Beschleunigungssensor,
- ein Temperatursensor,
- ein akustischer Sensor,
- ein Ultraschallsensor,
- ein Sauerstoffsensor,
- ein Drucksensor, und/oder
- ein Magnetfeldsensor.

Zusätzliche Sensoren können, gemäß einer Ausführungsform der vorliegenden Erfindung, mit der Auswerteeinheit gekoppelt sein. Die Auswerteeinheit kann die Sensordaten beispielsweise hinsichtlich verschiedener Parameter auswerten, die einen physiologischen Zustand des Patienten und/oder einen Zustand der Stimulationsvorrichtung selbst repräsentieren. Beispielhafte Parameter stellen dar: Erfolg einer Stimulationstherapie, Bedarfszustand einer Stimulationstherapie, ein gesundheitlicher Zustand des Patienten, ein Zustandsparameter der Umgebung der Stimulationsvorrichtung, etc.

Gemäß eines weiteren Aspekts der vorliegenden Erfindung ist die Stimulationsvorrichtung dazu ausgelegt, eine Stimulation durch die elektromagnetischen Wellen an Herzgewebe abzugeben. Dabei wird die Stimulation zeitlich relativ zum einem Herzzyklus ausgeführt. Beispielsweise kann in einem den Herzzyklus repräsentierenden Signal ein charakteristisches Ereignis gemessen werden (e.g. eine bradykarde/tachykarde Episode, eine Auffälligkeit im Signalverlauf etc.), woraufhin ein Zähler gestartet wird. Wird der Ablauf eines Zeitintervalls durch den Zähler festgestellt, wird die Stimulation ausgeführt. Die Stimulation ist dazu ausgelegt, dass zumindest eine Kontraktion des Herzens bewirkt wird.

Gemäß einer Ausführungsform der Erfindung wird dabei die Stimulation in Form von mindestens einem Stimulus abgegeben, wenn die Auswerteeinheit ein Therapieerfordernis für eine Bradykardie detektiert, wobei die Detektion auf der Unterschreitung mindestens einer festgelegten Herzfrequenz durch die gemessene Herzfrequenz basiert, und/oder
wenn die Auswerteeinheit ein Therapieerfordernis für eine ventrikuläre Fibrillation detektiert, wobei die Detektion auf dem Vergleich mindestens einer festgelegten ventrikulären Frequenz und/oder einer festgelegten Stabilität einer ventrikulären Frequenz durch die gemessene ventrikuläre Frequenz basiert.
In dieser Ausführungsform ist die Stimulationsvorrichtung ausgelegt zur Therapie von Bradykardien oder ventrikulärer Fibrillation.

Gemäß einem Aspekt der Erfindung wird dabei die Stimulation in Form von mehreren Stimuli abgegeben, wenn die Auswerteeinheit ein Therapieerfordernis für eine Tachykardie detektiert, wobei die Detektion auf der Überschreitung mindestens einer festgelegten Herzfrequenz durch die gemessene Herzfrequenz basiert, wobei mehrere Stimuli in einer Sequenz abgegeben werden, und/oder
wenn die Auswerteeinheit ein Therapieerfordernis für eine kardiale Resynchronisation detektiert, wobei die Detektion auf der Auswertung von Messdaten basiert, die die Herzaktivität repräsentieren, wobei mindestens ein erster und ein zweiter Stimulus abgegeben werden und wobei der mindestens erste und zweite Stimulus dazu ausgelegt sind, eine im Wesentlichen synchrone Kontraktion einer linken und einer rechten Herzhälfte zu erwirken.

Gemäß einem Aspekt der Erfindung wird dabei die Stimulation in Form von mehreren Stimuli abgegeben, wenn die Auswerteeinheit ein Therapieerfordernis für eine Tachykardie detektiert, wobei die Detektion auf der Überschreitung mindestens einer festgelegten Herzfrequenz durch die gemessene Herzfrequenz basiert, und die Stimuli in einer der Sequenz abgegeben werden und ausgelegt sind, die Tachykardie zu terminieren. Dabei handelt es sich um eine atriale und/oder ventrikuläre Tachykardie oder um atriale Fibrillation. Die Detektion von atrialer Fibrillation basiert dabei beispielsweise auf dem Vergleich mindestens einer festgelegten atrialen Frequenz und/oder einer festgelegten Stabilität einer atrialen Frequenz mit der gemessenen atrialen Frequenz. Die Stimuli der Sequenz sind gemäß einem Ausführungsbeispiel dazu ausgelegt, die atriale Fibrillation zu terminieren.

In den zwei zuvor genannten Ausführungsformen ist die Stimulationsvorrichtung ausgelegt zur Therapie einer Tachykardie oder Therapie einer Herzinsuffizienz verursacht durch die Asynchronizität zwischen der Kontraktion der rechten und linken Herzhälfte.

Gemäß einem Aspekt der Erfindung wird dabei die Stimulation in Form von mindestens einem Stimulus abgegeben, wenn die Auswerteeinheit ein Therapieerfordernis für eine ventrikuläre Fibrillation detektiert. Die Detektion der Therapieerfordernis basiert dabei auf dem Vergleich mindestens einer festgelegten ventrikulären Frequenz und/oder einer festgelegten Stabilität einer ventrikulären Frequenz durch die gemessene ventrikuläre Frequenz basiert. Der mindestens eine abgegebene Stimulus ist dazu ausgelegt, die ventrikuläre Fibrillation zu terminieren.

Des Weiteren, nach einem Aspekt der vorliegenden Erfindung, umfasst die Stimulationsvorrichtung eine oder mehrere Aufnahmeeinheiten in einer oder mehreren Herzkammern. Beispielsweise können Aufnahmeeinheiten im rechten Atrium oder rechten Ventrikel angeordnet sein. Die Aufnahmeeinheiten sind vorzugsweise so angeordnet, dass sie Signale aus dem rechten Atrium, rechten Ventrikel, linken Atrium, und/oder linken Ventrikel empfangen können. Die Signale können beispielsweise elektromagnetischer, elektrischer oder akustischer Natur sein.

Nach einem weiteren Ausführungsbeispiel der vorliegenden Erfindung wird der Stimulus oder die Stimuli durch die Stimulationsvorrichtung relativ zum einem Herzzyklus nach Ablauf eines Zeitintervalls abgegeben. Dabei weist Steuereinheit eine Zeitgebereinheit oder Zähler auf, die das Zeitintervall starten oder beenden kann. Das Starten und Beenden des Zeitintervalls wird durch die Aufnahmeeinheit ausgelöst, wobei nach Ablauf des Zeitintervalls eine Stimulation ausgelöst wird.

Gemäß der Erfindung wird ein Verfahren zur Ansteuerung einer implantierbaren Stimulationsvorrichtung vorgeschlagen, das zumindest die Schritte umfasst:
Veranlassung der Stimulationsvorrichtung zur Aussendung elektromagnetischer Wellen,
sodass eine Stimulation von genetisch manipuliertem Gewebe durch besagte elektromagnetische Wellen ausgelöst wird.

Die zuvor genannten Aspekte und Ausführungsformen der erfindungsgemäßen Stimulationsvorrichtung und der umfassten Komponenten sind ebenso anzuwenden auf das Verfahren zur Ansteuerung derselben sowie Ansteuerung der durch die erfindungsgemäße Stimulationsvorrichtung umfassten Komponenten.

Des Weiteren, nach einem weiteren Aspekt umfasst die erfindungsgemäße Stimulationsvorrichtung einen implantierbaren Stimulator ohne langgestreckte Elektrodenleitung. Die häufig im Zusammenhang mit elektrischer Stimulation eingesetzten Elektroden- bzw. Sondenleitungen können eine zusätzliche Komplikationsquelle für Infektionen oder Leitungsbrüche bzw. -kurzschlüsse darstellen.

Gemäß eines Aspekts der Erfindung umfasst die Stimulationsvorrichtung zumindest:
- eine Energiequelle,
- eine Elektronikeinheit, wobei die Elektronikeinheit eine Steuereinheit umfasst,
- einen Aktuator, der mit der Elektronik und/oder der Energiequelle gekoppelt ist,
- ein Gehäuse, wobei die Energiequelle, die Elektronikeinheit und der Aktuator im Gehäuse angeordnet sind,
- eine Fixiereinheit, die mit dem Gehäuse gekoppelt ist, wobei die Fixiereinheit dazu ausgelegt ist, die Stimulationsvorrichtung an einem Herzen oder in einem Herzen zu fixieren. Der Aktuator ist dazu ausgelegt, elektromagnetische Wellen zur Stimulation von genetisch manipuliertem Gewebe auszusenden. Die Steuereinheit ist dazu ausgelegt, die Stimulation des Gewebes mittels der elektromagnetischen Wellen des Aktuators zu steuern.

Des Weiteren, nach einem Ausführungsbeispiel der vorliegenden Erfindung, weist die Elektronikeinheit mindestens eine der folgenden oder eine Kombination aus den folgenden Einheiten auf:
eine Aufnahmeeinheit ausgelegt zur Messung von Daten, die die Gewebeaktivität und/oder Erfolg einer Stimulation charakterisieren, und/oder
eine Auswerteeinheit zur Auswertung von Messdaten hinsichtlich des Erfordernisses einer Stimulation und/oder hinsichtlich des Erfolgs einer Stimulation.

Gemäß einer Ausführungsform der vorliegenden Erfindung ist der Aktuator mindestens teilweise mit einem bio-kompatiblen Material beschichtet. Insbesondere wenn der Aktuator außerhalb des Gehäuses angeordnet ist und in direktem Kontakt mit der Umgebung, d.h. im implantierten Zustand in Kontakt mit Körperflüssigkeiten, erfordert der Aktuator eine derartige biokompatible Beschichtung. Nach einem Aspekt der erfindungsgemäßen Stimulationsvorrichtung ist der Aktuator dazu ausgelegt, einen lokalen Bereich am oder im Herzen mittels elektromagnetischer Wellen zu stimulieren.

Vorzugsweise, nach einem Ausführungsbeispiel der vorliegenden Erfindung, weist die Stimulationsvorrichtung mindestens einen ersten und einen zweiten Aktuator auf, die dazu ausgelegt sind, unterschiedliche lokale Bereiche am oder im Herzen mittels elektromagnetischer Wellen zu stimulieren.
Ein zweiter Aktuator kann mit der Elektronikeinheit und/oder Steuereinheit der Stimulationsvorrichtung verbunden sein und von denselben angesteuert werden.

Gemäß eines Ausführungsbeispiels ist/sind die Fixiereinheit und/oder zumindest Teile des Gehäuses mit einer entzündungshemmenden Medikation versehen, z.B. einem Steroid.

Auch denkbar wäre es, dass die erfindungsgemäße Stimulationsvorrichtung über eine Einheit zur elektrischen Stimulation des Herzens verfügt. Die Stimulationsvorrichtung könnte so angesteuert werden, dass sie mittels elektromagnetischen Wellen oder mittels galvanisch eingekoppelter Ströme eine Stimulation des Gewebes auslöst, oder durch eine Kombination der beiden. Elektromagnetische Wellen würden durch den Aktuator ausgesendet werden, während galvanische Ströme durch die Einheit zur elektrischen Stimulation des Herzens eingekoppelt werden.

Gemäß einem Aspekt der vorliegenden Erfindung umfasst die Stimulationsvorrichtung:
- eine Energiequelle,
- eine Elektronikeinheit, wobei die Elektronikeinheit eine Steuereinheit umfasst,
- einen Aktuator, der mit der Elektronik und/oder der Energiequelle gekoppelt ist, und wobei der Aktuator ausgelegt ist, elektromagnetische Wellen zur Stimulation von genetisch manipuliertem Gewebe auszusenden,
eine Fixiereinheit,
wobei die Stimulationsvorrichtung zur mindestens temporären Implantation im menschlichen oder tierischen Körper ausgelegt ist, und die Steuereinheit dazu ausgelegt ist, die Stimulation besagten Gewebes mittels der elektromagnetischen Wellen des Aktuators zu steuern. Dazu weist die Stimulationsvorrichtung eine Kontrolleinheit auf, die Daten aufnimmt, die die Gewebeaktivität und/oder Erfolg einer Stimulation durch eine über den elektromagnetischen Aktuator durchgeführte Stimulation charakterisieren.

Des Weiteren, nach weiteren Ausführungsbeispielen der vorliegenden Erfindung, nimmt die Kontrolleinheit besagte Daten auf Grundlage von mindestens einem oder einer Kombination als folgenden Signalen auf:
- ein elektrisch abgeleitetes Fernfeldsignal,
- Herztöne,
- ein Drucksignal,
- ein optisches Signal nach dem Prinzip der Pulsoxymetrie,
- ein Ultraschallsignal,
- ein Beschleunigungssignal, und/oder
- ein thermisches Signal.

Die Stimulationsvorrichtung ist gemäß einem Ausführungsbeispiel dazu ausgelegt, ein Ultraschallsignal auf Grundlage der Doppler-Technik aufzunehmen und auszuwerten, um somit auf Fließgeschwindigkeiten und -richtungen von Körperflüssigkeiten schließen zu können.

Gemäß einem Ausführungsbeispiel der erfindungsgemäßen Stimulationsvorrichtung steht die Kontrolleinheit nicht in elektrischem Kontakt mit dem genetisch manipulierten Gewebe. Die Kontrolleinheit ist derart angeordnet, dass die auszuwertenden Signale, die die Gewebeaktivität und/oder Erfolg einer Stimulation mittels des Aktuators charakterisieren, direkt von der Kontrolleinheit durch Sensoren aufgenommen werden kann. Alternativ können die Sensoren von der Kontrolleinheit separiert angeordnet sein. Dann werden die von den Sensoren aufgenommenen Signale zur Kontrolleinheit weitergeleitet. Die Kontrolleinheit kann Teil der Elektronikeinheit bilden.

Gemäß eines Ausführungsbeispiels der vorliegenden Erfindung wird ein Stimulationssystem beschrieben, das zumindest umfasst:
- eine Energiequelle,
- eine Elektronikeinheit, wobei die Elektronikeinheit eine Steuereinheit umfasst,
- einen Aktuator, der mit der Elektronik und/oder der Energiequelle gekoppelt ist, und wobei der Aktuator ausgelegt ist, elektromagnetische Wellen zur Stimulation von genetisch manipuliertem Gewebe auszusenden,
- ein Gehäuse, in dem zumindest die Elektronikeinheit angeordnet ist,
wobei die Stimulationssystem zur mindestens temporären Implantation im menschlichen oder tierischen Körper ausgelegt ist, und die Steuereinheit dazu ausgelegt ist, die Stimulation besagten Gewebes mittels der elektromagnetischen Wellen des Aktuators zu steuern. Außerdem umfasst das Stimulationssystem ein Selektionsmittel, das dazu ausgelegt ist, den Bereich oder Areal des besagten Gewebes für die Stimulation zu wählen.

Gemäß eines Aspekts des erfindungsgemäßen Stimulationssystems ist der Aktuator dazu ausgelegt, die elektromagnetischen Wellen in mindestens einer Abstrahlrichtung auszusenden, wobei die Selektionsmittel dazu ausgelegt ist, die Abstrahlrichtung zu steuern.

Nach einem Ausführungsbeispiel sendet der Aktuator elektromagnetische Wellen in einem Raumwinkel von weniger als 4*π aus.

Des Weiteren, nach einem Ausführungsbeispiel des erfindungsgemäßen Stimulationssystems weist das Selektionsmittel mindestens ein Maskierungsmittel auf, das dazu ausgelegt ist, ein Areal besagten Gewebes zu maskieren, sodass die Intensität der Stimulation für das Areal reduziert oder gleich Null ist.

Nach einem Ausführungsbeispiel ist das Maskierungsmittel dazu ausgelegt, einen Raumwinkel, mit dem die elektromagnetischen Wellen durch den Aktuator ausgesendet werden, zu verändern.

Nach einem Ausführungsbeispiel umfasst das Maskierungsmittel mindestens ein Filter, wobei das Filter
- elektromagnetische Strahlung bestimmter Frequenzbereiche sperrt, oder
- elektromagnetische Strahlung bestimmter Polarisationsrichtungen sperrt.

Nach einem Ausführungsbeispiel ist die Maskierungsvorrichtung mit dem Aktuator verbunden oder ist an den Aktuator fixiert ist. Alternativ kann sie mit dem Aktuator eine Einheit bilden.

Nach einem Ausführungsbeispiel steht die Maskierungsvorrichtung mit dem Areal des besagten Gewebes in Kontakt stehen. Ein konkretes Ausführungsbeispiel bestünde darin, dass die Maskierungsvorrichtung als Deckschicht (z.B. für elektromagnetische Strahlung undurchlässige Farbe) oder Deckvorrichtung ausgeführt ist, die besagte Gewebsareale abdeckt. Des Weiteren, nach einem Ausführungsbeispiel der vorliegenden Erfindung, wird die Maskierungsvorrichtung durch einen Teil des Gehäuses gebildet. Dies kann beispielsweise dadurch realisiert werden, in dem die Maskierungsvorrichtung am Gehäuse angebracht ist (z.B. in Form von Blenden).

Des Weiteren, gemäß eines Ausführungsbeispiels, ist der Aktuator derart angeordnet, dass im implantierten Zustand des Stimulationssystems ein Umgebungsobjekt zur Maskierung von mindestens einem Areal des besagten Gewebes dient, sodass die Intensität der Stimulation für das Areal reduziert oder gleich Null ist. Beispielsweise kann hierzu der Aktuator in einer Kavität in der Umgebung des Implantationsortes angeordnet sein, sodass die ihn umgebende Gewebetopologie als natürliche Maskierung für die ausgesendeten elektromagnetischen Wellen dient. Wird das Stimulationssystem im Herzen implantiert, so kann der Aktuator im Herzohr implantiert werden, das sich im rechten Atrium befindet. So wird verhindert, dass elektromagnetische Wellen in den in den rechten Ventrikel eingestrahlt werden kann, d.h. eine selektive Einstrahlung für das Atrium wird ermöglicht. Wird in einem weiteren Beispiel eine Implantation des Aktuators unterhalb des Moderatorbandes vorgenommen, so können keine elektromagnetischen Wellen in das Atrium eingestrahlt werden, d.h. selektive Einstrahlung in den rechten Ventrikel wird ermöglicht.

Gemäß eines weiteren Aspekts des erfindungsgemäßen Stimulationssystem ist das Areal für die Bestrahlung des besagten Gewebes durch Anpassung des Maskierungsmittels veränderbar. Beispielsweise kann auf Grundlage von Messungen des Stimulationserfolgs eine Nachbesserung der Behandlung der Zellstrukturen erforderlich sein. Dabei kann das Areal für die Bestrahlung gegebenenfalls durch zusätzliche Behandlung erweitert oder durch Verwendung des Maskierungsmittels verkleinert werden.

Gemäß einer Ausführungsform des erfindungsgemäßen Stimulationssystems weist das Selektionsmittel eine Trägerstruktur auf, die mit dem Gehäuse verbunden ist, wobei der Aktuator mit der Trägerstruktur verbunden ist. Die Trägerstruktur kann so ausgelegt sein, dass das Selektionsmittel und/oder der Aktuator an verschiedenen Stellen der Trägerstruktur angeordnet und fixiert werden kann. Auf diese Weise kann man die räumliche Anordnung zwischen Selektionsmittel und Aktuator ändern und somit den Abstrahlwinkel des Aktuators flexibler anpassen. Gemäß einem Ausführungsbeispiel ist die Trägerstruktur ausgelegt, den Abstrahlwinkel der ausgesendeten elektromagnetischen Wellen durch den Aktuator einzuschränken.

Des Weiteren, nach einem Aspekt des erfindungsgemäßen Stimulationssystems weist das Stimulationssystem mindestens einen ersten und einen zweiten Aktuator auf. Die Aktuatoren sind vorzugsweise so angeordnet, dass bei gleichzeitigem Aussenden elektromagnetischer Wellen Interferenzen minimiert werden. So können zwei Gewebsareale unabhängig voneinander bestrahlt werden. Nach einem Aspekt der Erfindung weisen die durch den ersten und zweiten Aktuator ausgesendeten elektromagnetischen Wellen unterschiedliche Frequenzen und/oder unterschiedliche Polarisation auf.

Des Weiteren, nach einem Ausführungsbeispiel des erfindungsgemäßen Stimulationssystems weist das Stimulationssystem ein Mittel zur Bündelung elektromagnetischer Strahlung auf. Ein Mittel zur Bündelung umfasst z.B.:
- Linsen,
- Kollimatoren,
- Vorrichtungen, die Materialien mit anisotropen Ausbreitungseigenschaften für die genutzte elektromagnetische Strahlung besitzen.

Die Erfindung ist nachfolgend beispielhaft, anhand von einem in Zeichnungen dargestellten Ausführungsbeispiel, näher erläutert. In den Figuren sind funktionell gleiche oder gleich wirkende Elemente jeweils mit denselben Bezugszeichen beziffert. Die Figur ist eine schematische Darstellung der Erfindung und bildet nicht spezifische Parameter der Erfindung ab. Die Figur gibt lediglich typische Ausgestaltungen der Erfindung wieder und soll die Erfindung nicht auf die dargestellten Ausgestaltungen beschränken.

Gemäß der Erfindung wird eine Vorrichtung vorgeschlagen, die mindestens umfasst:
- eine Energiequelle,
- einen Energiespeicher,
- eine Elektronikeinheit,
- wobei die Vorrichtung zur Implantation im menschlichen oder tierischen Körper ausgelegt ist,
- und einen Aktuator, der mit dem Energiespeicher gekoppelt ist und dazu ausgelegt ist, durch Entladung des Energiespeichers elektromagnetische Wellen auszusenden.

Des Weiteren, nach einem Aspekt der vorliegenden erfinderischen Vorrichtung, weist der Energiespeicher einen Kondensator und/oder eine Spule auf.

Nach einem Aspekt der vorliegenden erfinderischen Vorrichtung umfasst die Elektronikeinheit eine Steuereinheit, wobei die Steuereinheit mindestens eine der folgenden Eigenschaften aufweist:
sie ist dazu ausgelegt, eine Ladung des Energiespeichers und Entladung des Energiespeichers an den Aktuator zu steuern,
- sie ist dazu ausgelegt, die Energiemenge für eine Ladung und Entladung zu steuern,
- sie ist mittels einer Programmiervorrichtung von extern konfigurierbar,
- sie weist eine Freigabeeinheit für die Entladung auf, und/oder
- die Entladung findet über mindestens zwei in Reihe geschaltete Impedanzen statt, wobei die Impedanzen mittels der Steuereinheit einstellbar sind.

Beispielsweise können solche Impedanzen durch elektrische Schalterelemente realisiert werden. Solche Schalterelemente ermöglichen so den Verlauf der Entladung.

Gemäß eines Ausführungsbeispiels der erfindungsgemäßen Vorrichtung wird die Entladung durch die Steuereinheit derart gesteuert, dass der Aktuator in einem Zeitraum von 0,1ms-5s elektromagnetische Wellen in Form eines durchgehenden Wellenzugs aussendet. Für eine Neurostimulationsanwendung (z.B. Rückenmarkstimulation, Vagusnerv-Stimulation), kardiales Pacing oder ATP-Stimulation kann ein Zeitraum von 0,1ms-2ms gewählt werden, 0.1s-5s für Kardioversion oder Defibrillation des Herzens.

Des Weiteren, nach einem Aspekt der erfindungsgemäßen Vorrichtung, findet die Entladung in mehr als einer Phase stattfindet.

Nach einem Ausführungsbeispiel der erfindungsgemäßen Vorrichtung weist der Aktuator mindestens eine der folgenden Eigenschaften auf:
- er umfasst mindestens eine Lichtquelle zur Aussendung der elektromagnetischen Wellen,
- er umfasst mindestens einen Strombegrenzer (z.B. Widerstand oder Diode),
- er wird durch den Energiespeicher mit 1V bis zu 1500 V betrieben (spezielle Lösungen wären z.B.: 1-10V für eine Parallelschaltung, 50-500 V für eine Reihenschaltung)
- und/oder
- er ist von einem Gehäuse der Vorrichtung getrennt angeordnet und verfügt über einen Steckverbinder, wobei der Steckverbinder kompatibel ist zu einem Steckerausgang eines implantierbaren Geräts zur elektrischen Herzstimulation.

Gemäß einem weiteren Aspekt der Erfindung umfasst die Lichtquelle eine Reihenschaltung von LEDs (Light Emitting Diode), eine Parallelschaltung von LEDs, oder eine Kombination aus Reihenschaltung und Parallelschaltung von LEDs. Die Verwendung von LEDs bietet u.a. die Vorteile, dass die Miniaturisierung der Lichtquelle auf einfache Art ermöglicht und der Energieverbrauch gering gehalten wird, besonders bei der Implementierung in einem Implantat entscheidende Faktoren sein können.

Gemäß einem weiteren Aspekt der Erfindung umfasst die Lichtquelle eine Laserlichtquelle, die Laserlicht mit einer für die Stimulation von genetisch manipuliertem Gewebe geeignete Wellenlänge aufweist. Laserlicht ermöglicht Stimulation mit sehr hoher örtlicher Präzision.

Gemäß einem Ausführungsbeispiel der vorliegenden Erfindung wird eine implantierbare Stimulationsvorrichtung zur Stimulation von Herzgewebe oder Nervengewebsstrukturen vorgeschlagen, die besagte erfindungsgemäße Vorrichtung aufweist. Das Gerät kann mindestens eine Stimulationselektrode aufweisen.

Nach einem Aspekt der erfindungsgemäßen Stimulationsvorrichtung ist diese dazu ausgelegt, Stimulation von Herzgewebe hervorzurufen, wobei die Stimulation
- mittels des Aktuators durch elektromagnetische Wellen oder
- mittels elektrischer Stimulation,
- wobei die Stimulation durch elektromagnetische Wellen und durch elektrische Stimulation einzeln, nacheinander oder zeitgleich stattfindet,
- wobei die Stimulation in einem Areal oder mehreren Arealen des Gewebes stattfindet.

Gemäß einem Ausführungsbeispiel der vorliegenden Erfindung umfasst die Stimulationsvorrichtung weiter:
- einen Steckkontakt kompatibel für einen Aktuator, und/oder
- einen Steckkontakt kompatibel für eine Elektrode für elektrische Stimulation, und/oder
- Aktuator und eine Elektrode.

Gemäß einem Ausführungsbeispiel der Stimulationsvorrichtung gibt sie über den Aktuator elektromagnetische Wellen zur Stimulation von besagtem manipulierten Gewebe ab, sie verfügt über eine Erfolgskontrolle und gibt im nicht erfolgreichen Fall eine zusätzliche elektrische Therapie ab. Beispielsweise kann es auch beide Therapiearten zugleich abgeben oder gezielt zwischen beiden umgeschaltet werden. An welchen Orten welche Therapieform eingesetzt wird, kann programmierbar sein oder/und wird anhand von einer Analyse der Therapieerfolge der Vergangenheit durch die Stimulationsvorrichtung selbst bestimmt und ggf. umgestellt. Vorzugsweise verfügt die erfindungsgemäße Stimulationsvorrichtung über dedizierte Anschlüsse für den elektromagnetischen Aktuator.

In einer bevorzugten Ausführung verfügt die erfindungsgemäße Stimulationsvorrichtung über mindestens einen Anschluss, der sowohl für einen Aktuator gemäß der Erfindung als auch für eine elektrische Stimulation nach Stand der Technik genutzt werden kann. Gemäß einer Ausführungsform ist der Aktuator mit der Stimulationsvorrichtung verbindbar über einen Stecker, der einem Stecker für eine vergleichbare Komponente für die elektrische Therapie entspricht (z.B. Stecker einer Elektrodenleitung für die kardiale Stimulation). Bevorzugt wird der Aktuator gemäß der Erfindung durch gleiche oder zumindest ähnliche Spannungen betrieben wie solche, die für die elektrische Therapie nach Stand der Technik genutzt werden (z.B. kardiale Schrittmachertherapie, Neurostimulatoren).

Gemäß einem Ausführungsbeispiel der Erfindung wird eine im menschlichen oder tierischen Körper implantierbare Vorrichtung vorgeschlagen, die mindestens eine Substanz umfasst. Die Substanz ist dazu ausgelegt, menschliche oder tierische Zellstrukturen zu modifizieren, sodass Aktionspotentiale in den Zellstrukturen durch Bestrahlung durch elektromagnetische Wellen im Frequenzbereich 10¹³-10²⁰ Hz wahrnehmbar und/oder evozierbar sind. Die Vorrichtung umfasst dabei ein Applikationsmittel zur Abgabe der Substanz an Gewebe.

Des Weiteren, nach einem Aspekt der vorliegenden Erfindung, umfasst das Applikationsmittel zur Abgabe der Substanz an Gewebe zumindest eines der folgenden Mittel:
- eine Kanüle,
- ein Mittel zum Sprühen, Aufpinseln, Träufeln und/oder Aufstempeln der Substanz.

Gemäß einem Ausführungsbeispiel der vorliegenden Erfindung weist die Vorrichtung mindestens eine Zuleitung für die Substanz auf. Auch in Betracht zu ziehen wäre es, wenn die Vorrichtung mindestens eine Konservierungsvorrichtung für die Substanz aufweist.

Nach einem Ausführungsbeispiel weist die Vorrichtung ein Reservoir auf, das zur Aufbewahrung der Substanz ausgelegt ist. Die Vorrichtung kann ein Gehäuse aufweisen, wobei das Reservoir innerhalb des Gehäuses oder außerhalb des Gehäuses angeordnet ist. Das Reservoir kann eine biokompatible Umhüllung, und/oder eine thermische Isolierung, und/oder einen Schutz gegen harte Strahlung aufweisen. Auf diese Weise kann die Substanz im Inneren des Reservoirs effektiv vor äußeren Einflüssen geschützt werden.

Nach einem Aspekt der erfindungsgemäßen Vorrichtung weist diese einen Port zum Auffüllen des Reservoirs auf. Der Port kann z.B. an der Zuleitung, am Gerätegehäuse, oder am Reservoir angebracht sein. In einem Beispiel umfasst der Port eine Membran. Die Membran kann mindestens eine der folgenden Eigenschaften aufweisen:
- sie ist so positioniert, dass sie mit einem Werkzeug zum Befüllen mit der Substanz zugänglich ist,
- sie ist mehrfach durchstechbar, und/oder
- sie ist so ausgelegt, dass sie nach Entfernen des Werkzeugs zum Befüllen wieder schließt, sodass ein Austreten der befüllten Substanz im Wesentlichen verhindert wird.

Gemäß einer Ausführungsform kann das Reservoir einen Teil der Zuleitung bilden.

Des Weiteren, nach einem Aspekt der erfindungsgemäßen Vorrichtung, weist die Konservierungsvorrichtung mindestens eines der folgenden Mittel auf:
- ein thermisches Element zur Kühlung und/oder Erhitzung,
- einen Generator für Strahlung zur Sterilisation,
- einen Speicher für eine Konservierungssubstanz, wobei die Konservierungssubstanz der Substanz zugebbar ist, und/oder
- ein Kontrollmittel zur Ermittlung eines Konservierungsstatus der Substanz.

Nach einem Ausführungsbeispiel umfasst die erfindungsgemäße Vorrichtung ein Regelmittel oder ist mit einem solchen verbindbar. Das Regelmittel ist dazu ausgelegt, einen Grad der Modifikation bezüglich Wahrnehmbarkeit und/oder Evozierbarkeit von Aktionspotentialen in Gewebe mittels elektromagnetischer Wellen zu ermitteln. Das Regelmittel kann mindestens eine der folgenden Eigenschaften aufweisen: bei Ermittlung eines niedrigen Grads wird der Bedarf einer Abgabe der Substanz durch das Applikationsmittel festgestellt, und/oder
die Ermittlung des Grads basiert auf Messdaten bezüglich einer Wahrnehmung und/oder Evozierung von Aktionspotentialen in Gewebe mittels elektromagnetischer Wellen im Frequenzbereich 10¹³-10²⁰ Hz.

Nach einem Beispiel umfasst die erfindungsgemäße Vorrichtung mindestens eines der folgenden Mittel:
ein Ventil zum Freisetzen der Substanz,
eine Pumpe, und/oder
eine Maskiervorrichtung, dazu ausgelegt, Gewebeareale zu maskieren, an die keine Substanz abgegeben werden soll.

Des Weiteren, nach einer Ausführungsform, umfasst die Vorrichtung mindestens:
- eine Energiequelle,
- eine Elektronikeinheit,
- einen Aktuator, der mit der Elektronikeinheit und/oder der Energiequelle gekoppelt ist und dazu ausgelegt ist, elektromagnetische Wellen zur Stimulation von durch die Substanz behandeltes Gewebe auszusenden.

Nach einem Aspekt ist die Abgabe der Substanz an das Gewebe durch das Applikationsmittel über einen Zeitraum kontrollierbar. Beispielsweise kann das Applikationsmittel die Abgabe der Substanz über einen Zeitraum kontrollieren, z.B. in dem die Abgabe in regelmäßigen Abständen zu vorbestimmten Dosen stattfindet. Alternativ kann das Applikationsmittel ein über einen längeren Zeitraum biologisch abbaubares Mittel umfassen, das die Substanz enthält. Durch den Kontakt mit Gewebe wird das biologisch abbaubare Mittel zusammen mit der Substanz zeitlich kontrolliert an das Gewebe abgegeben. In einem Beispiel ist die Substanz selbst über einen längeren Zeitraum abbaubar.

Gemäß einem Ausführungsbeispiel wird eine Vorrichtung vorgeschlagen, die mindestens umfasst:
- eine Energiequelle,
- eine Elektronikeinheit,
- eine Aufnahmeeinheit, mit der Elektronikeinheit gekoppelt und dazu ausgelegt, elektromagnetische Wellen im Frequenzbereich 10¹³-10²⁰ Hz zu messen,
- wobei die Vorrichtung zur Implantation im menschlichen oder tierischen Körper ausgelegt ist. Die Vorrichtung ist dazu ausgelegt, zu detektieren, dass die elektromagnetischen Wellen von genetisch manipuliertem Gewebe abgestrahlt wurden.

Gemäß einem Aspekt der erfindungsgemäßen Vorrichtung weist diese einen Aktuator auf, der mit der Elektronikeinheit und/oder der Energiequelle gekoppelt ist und wobei der Aktuator dazu ausgelegt ist, elektromagnetische Wellen im Frequenzbereich 10¹³-10²⁰ Hz auszusenden.

Nach einem Aspekt der erfindungsgemäßen Vorrichtung ist die Elektronikeinheit dazu ausgelegt ist, zu erkennen, dass durch die Aufnahmeeinheit gemessene elektromagnetische Wellen vom Aktuator ausgesendet wurden. Beispielsweise kann es sich dabei um vom Aktuator ausgesendete Wellen handeln, die an Gewebe reflektiert werden. Die reflektierten Wellen werden von der Aufhahmeeinheit detektiert, und von der Elektronikeinheit entsprechend erkannt.

Zum Beispiel, gemäß einem Aspekt der erfindungsgemäßen Vorrichtung, handelt es sich bei der durch die Aufhahmeeinheit gemessenen elektromagnetischen Welle um die durch den Aktuator ausgesendete elektromagnetische Welle in veränderter Form. Die Veränderung beruht z.B. auf mindestens einem der Effekte:
- Reflexion,
- Fluoreszenz,
- Absorption,
- Transmission, und/oder
- Polarisation.

In einem Beispiel ist die Elektronikeinheit dazu ausgelegt, in der von der Aufhahmeeinheit gemessenen elektromagnetischen Welle mindestens einen der folgenden Parameter, eine Kombination aus diesen oder eine abgeleitete Größe aus diesen zu erkennen:
- Amplitude,
- Frequenz oder Frequenzspektrum, und/oder
- Polarisationsrichtung,
- Phase,
- wobei mindestens eines der folgenden Verfahren angewendet wird:
- Modulationsverfahren
- Pulsweitenmodulation (z.B. im Fall wenn die eingestrahlte elektromagnetische Welle durch die Gewebsstrukturen in Pulsen mit messbaren Pulsdauern umgesetzt wird, wobei aus den Pulsdauern Informationen über die Stimulation und/oder die Gewebseigenschaften gewonnen werden können),
- Erkennung der Veränderung durch Anwendung von Filtern (z.B. zum Detektieren des Frequenzspektrums der gemessenen elektromagnetischen Wellen).

Gemäß einem speziellen Ausführungsbeispiel erkennt die Elektronikeinheit die Frequenz bzw. eines Frequenzspektrums oder einer Frequenzverschiebung der von der Aufhahmeeinheit gemessenen elektromagnetischen Wellen über die Detektion der Veränderung infolge einer Filterwirkung.

Gemäß einem Beispiel ist die Elektronikeinheit dazu ausgelegt, in den von der Aufhahmeeinheit gemessenen elektromagnetischen Wellen die spektrale Leistungsdichte zu ermitteln.

Des Weiteren, nach einem Aspekt der erfindungsgemäßen Vorrichtung, wandelt die Elektronikeinheit oder Aufnahmeeinheit die gemessene elektromagnetische Welle in ein elektronisches Signal um und verarbeitet sie mittels mindestens eines der folgenden Verfahren vor:
- Verstärkung,
- Demodulation,
- Filterung,
- AD-Wandlung,
- Gleichrichtung,
- Bestimmung der Signalleistung,
- Schwellwertbestimmung,
- Transformation in den Frequenzbereich (z.B. via Fourier-, Hartley- oder Wavelet-Transformation)
- Bestimmung von Signalqualität (z.B. über Bestimmung des Signal-to-Noise-Ratio SNR), und/oder
- Bestimmung signalmorphologischer Parameter (z.B. Signalamplitude, Signalzacken, Verhältnisse von Signalamplituden und/oder Signalzacken etc.).

Nach einem Ausführungsbeispiel der erfindungsgemäßen Vorrichtung wandelt die Elektronikeinheit oder Aufnahmeeinheit die gemessene elektromagnetische Welle in ein elektronisches Signal. Des Weiteren führt die Elektronikeinheit eine Analyse des Signals auf Grundlage von mindestens einem der folgenden Verfahren durch:
- Segmentierung,
- Ereigniserkennung,
- Bestimmung von Periodizität,
- Bestimmung der Phasenlage (z.B. wird die Phasenlage zwischen besagtem Signal und einem anderen aufgenommenen Signal oder einem Referenzsignal bestimmt),
- Bestimmung von Stabilität (z.B. wird die Stabilität der Signalintensität oder die Stabilität eines in dem Signal detektierten Rhythmus erkannt), oder
- Klassifikation von Rhythmen, und/oder
- Klassifikation von signalmorphologischen Parametern.

Gemäß einem Ausführungsbeispiel der erfindungsgemäßen Vorrichtung weist die Aufnahmeeinheit mindestens einen der folgenden Sensoren auf:
- Photodiode,
- Phototransistor,
- Charge-Coupled Device (CCD)- Element, und/oder
- analoger oder digitaler Bildsensor.

Des Weiteren, gemäß einem Aspekt der vorliegenden Erfindung, umfasst die Vorrichtung mindestens einen ersten und einen zweiten Aktuator, wobei der erste und der zweite Aktuator elektromagnetische Wellen in unterschiedlichen Frequenzen aussenden. Der erste Aktuator kann mit einem Gehäuse der Vorrichtung gekoppelt sein. Alternativ kann der erste Aktuator entfernt vom Gehäuse angeordnet sein. Der erste Aktuator kann mit der Aufnahmeeinheit verbunden sein.

Nach einem Ausführungsbeispiel verfügt die Vorrichtung über ein Elektrostimulationsmittel. Die Vorrichtung ist nach einem Aspekt der Erfindung zum Aussenden elektromagnetischer Wellen an Herzgewebe oder an Nervengewebe im Rückenmark oder Muskelgewebe ausgelegt.

Gemäß Ausführungsbeispielen umfasst der zumindest erste Aktuator LEDs. In einer besonderen Realisierung umfasst der Aktuator ein Filter (z.B. Polarisationsfilter). Die Abstrahlcharakteristik (d.h. Richtung, Intensität, Frequenz, Dauer der Abstrahlung) ist gemäß einem Ausführungsbeispiel programmierbar durch die Steuereinheit. Handelt es sich bei der Vorrichtung um ein Implantat, kann besagte Programmierung vor und auch nach der Implantation erfolgen.

Gemäß einem Ausführungsbeispiel umfasst die Vorrichtung eine Fixiervorrichtung. Die Fixiervorrichtung ist so ausgebildet, dass Aktuatoren und/oder Sensoren daran derart befestigt sind, dass die elektromagnetische Strahlung das Gewebe durchdringt.

Eine Implantation der erfindungsgemäßen Vorrichtung kann erfolgt mittels einer steuerbaren Positionierhilfe (z.B. steuerbarer Katheter, Steerable Sheaths, over-the-wire OTW Technik etc.) erfolgen. Die Positionierung der Vorrichtung kann unter Verwendung von bildgebenden Verfahren (z.B. Röntgenbildgebung, Computertomographie, Magnetresonanztomographie, Ultraschall, Impedanztomographie) erfolgen.

Im Sinne der erfindungsgemäßen Idee wird eine Methode zur Ansteuerung einer im menschlichen oder tierischen Körper implantierbaren Vorrichtung vorgeschlagen. Die Methode weist mindestens die Schritte auf:
Veranlassung einer Messung elektromagnetischer Wellen im Frequenzbereich 10¹³-10²⁰ Hz, und
Veranlassung einer Detektion, ob die elektromagnetischen Wellen von genetisch manipuliertem Gewebe abgestrahlt wurden.
   - Figur 1: Schematische Darstellung eines Ausführungsbeispiels der erfindungsgemäßen Stimulationsvorrichtung im implantierten Zustand mit einem externen Gerät
   - Figur 2: Schematische Darstellung einer Methode zur Behandlung einer Bradykardie mittels optischer Stimulation für mindestens ein Ausführungsbeispiel der erfindungsgemäßen Stimulationsvorrichtung
   - Figur 3: Schematische Darstellung einer Methode zur Behandlung einer tachykarden Herzrhythmusstörung in Form einer ventrikulären oder atrialen Tachykardie für mindestens ein Ausführungsbeispiel der erfindungsgemäßen Stimulationsvorrichtung
   - Figur 4: Schematische Darstellung einer Methode zur Behandlung eines Vorhofflimmerns für mindestens ein Ausführungsbeispiel der erfindungsgemäßen Stimulationsvorrichtung
   - Figur 5: Schematische Darstellung einer Methode zur Behandlung eines Kammerflimmerns für mindestens ein Ausführungsbeispiel der erfindungsgemäßen Stimulationsvorrichtung
   - Figur 6: Schematische Darstellung einer Methode zur Anwendung der kardialen Resynchronisationstherapie mittels elektromagnetischer Wellen für mindestens ein Ausführungsbeispiel der erfindungsgemäßen Stimulationsvorrichtung
   - Figur 7: Schematische Darstellung einer Neurostimulationsmethode mittels elektromagnetischer Wellen für mindestens ein Ausführungsbeispiel der erfindungsgemäßen Stimulationsvorrichtung
   - Figur 8: Schematische Darstellung einer Ausführungsform eines erfindungsgemäßen implantierbaren Stimulators
   - Figur 9: Implantierbarer Stimulator gemäß Figur 8 mit einer alternativen Fixiervorrichtung
   - Figur 10: Blockschaltbild des implantierbaren Stimulators gemäß einem Ausführungsbeispiel der Erfindung

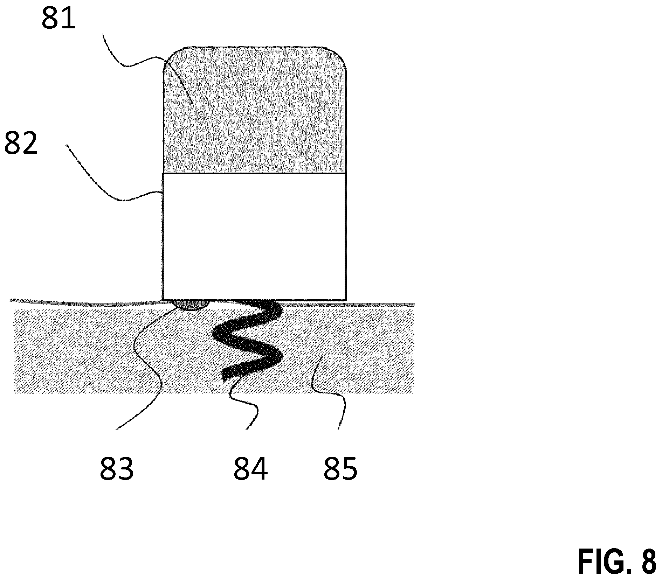

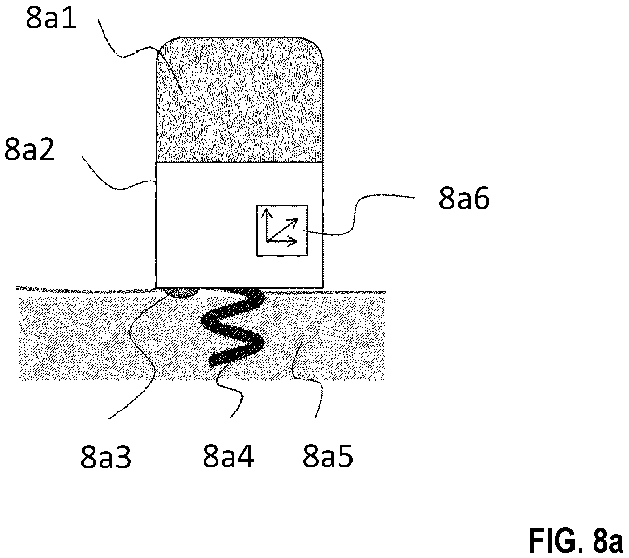

   - Figur 8a: Schematische Darstellung einer Ausführungsform der erfindungsgemäßen Stimulationsvorrichtung mit Therapieerfolgskontrolle

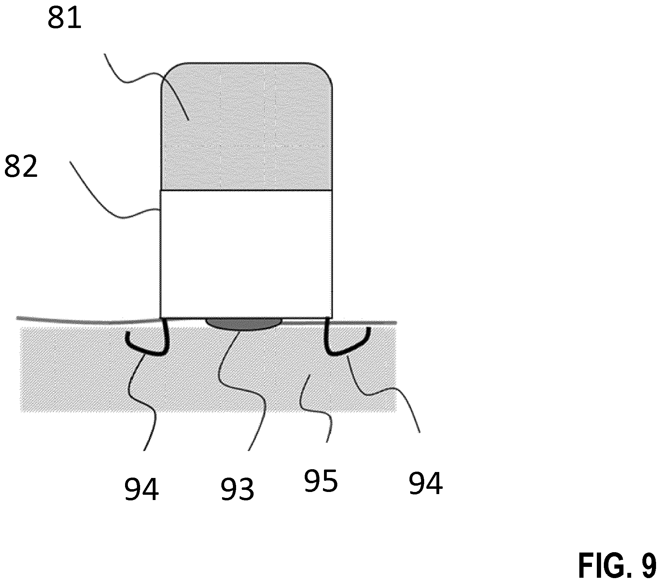

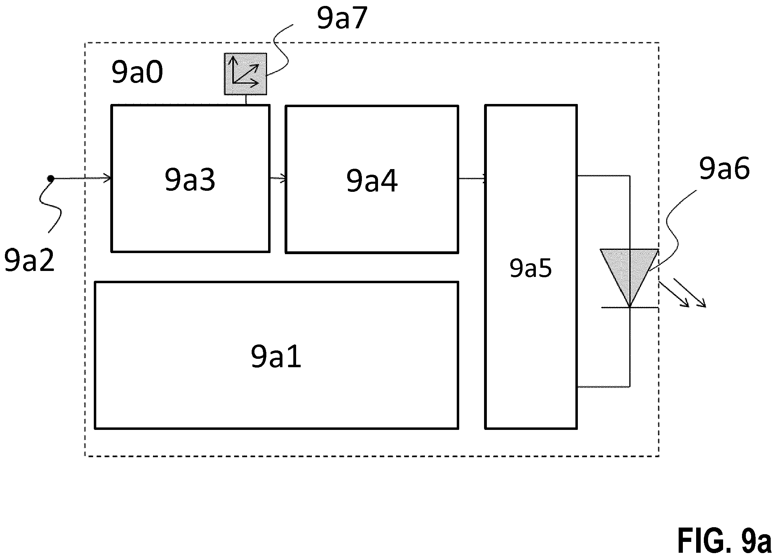

   - Figur 9a: Blockschaltbild der erfindungsgemäßen Stimulationsvorrichtung gemäß eines Ausführungsbeispiels

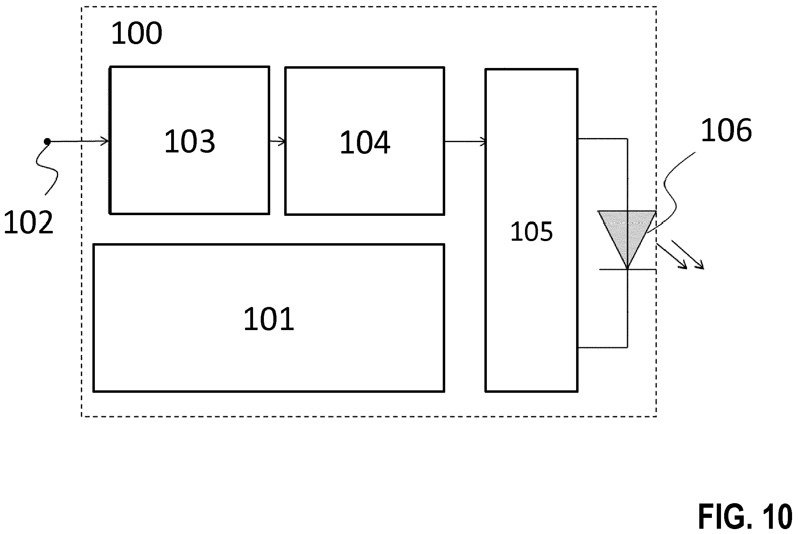

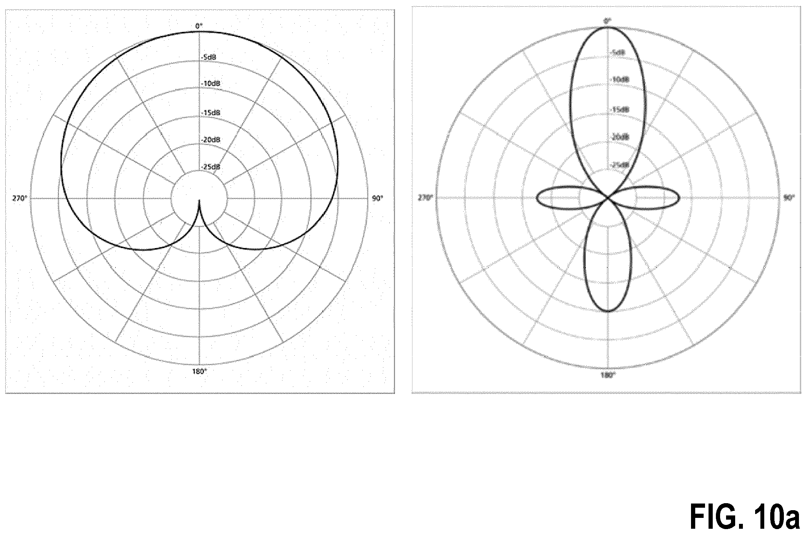

   - Figur 10a: zeigt beispielhaft zweidimensionale Charakteristiken elektromagnetischer Strahlers mit räumlich selektiver Wirkung.

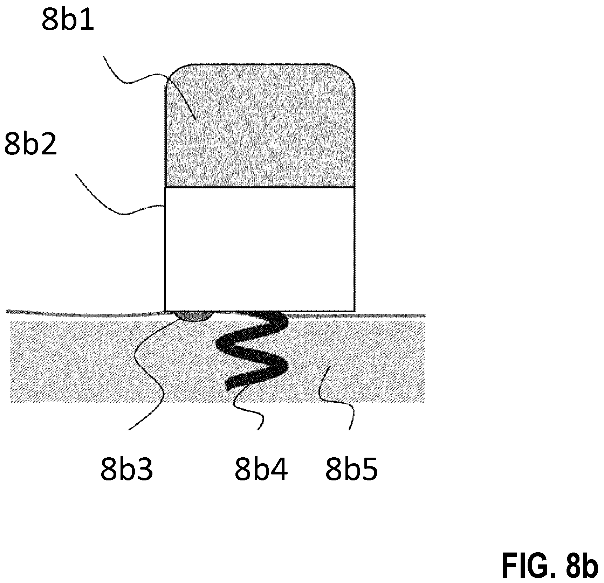

   - Figur 8b: Schematische Darstellung einer Ausführungsform der erfindungsgemäßen Stimulationsvorrichtung mit Defibrillationsfunktion

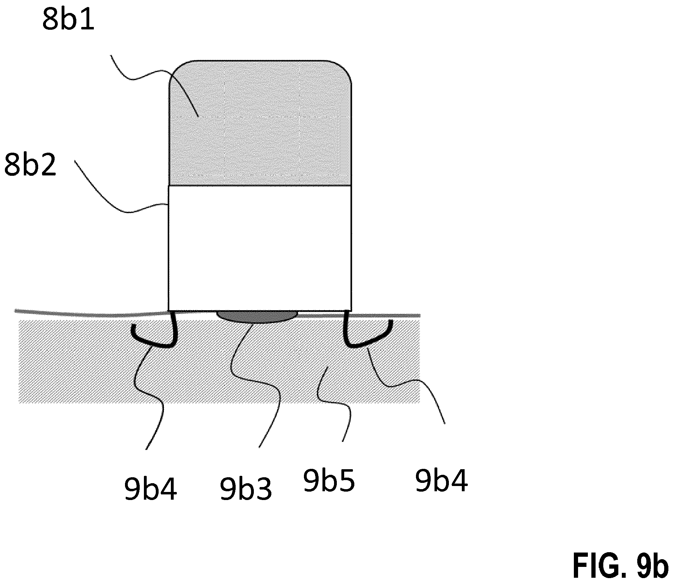

   - Figur 9b: Implantierbarer Stimulator gemäß Figur 8b mit einer alternativen Fixiervorrichtung
   - Figur 10a: Blockschaltbild der erfindungsgemäßen Stimulationsvorrichtung gemäß eines Ausführungsbeispiels nach Figur 8b

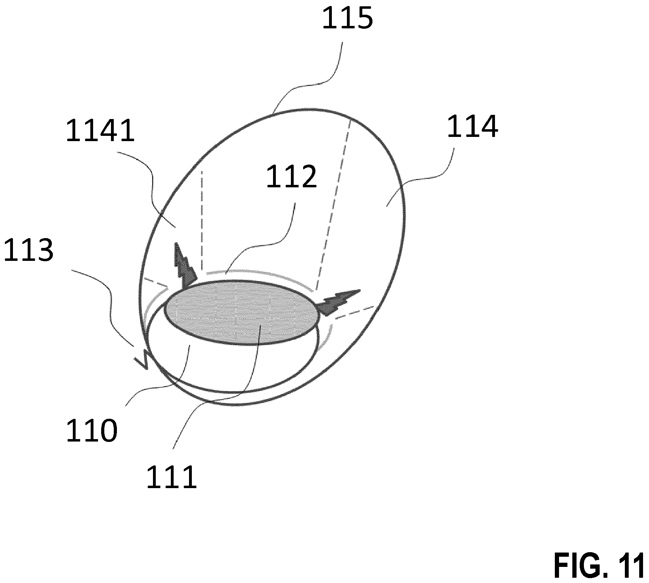

   - Figuren 11 bis 14: zeigen Realisierungen des erfindungsgemäßen Stimulationssystems mit Selektionsmittel, das dazu ausgelegt ist, den Bereich des besagten Gewebes für die Stimulation zu wählen gemäß Ausführungsformen

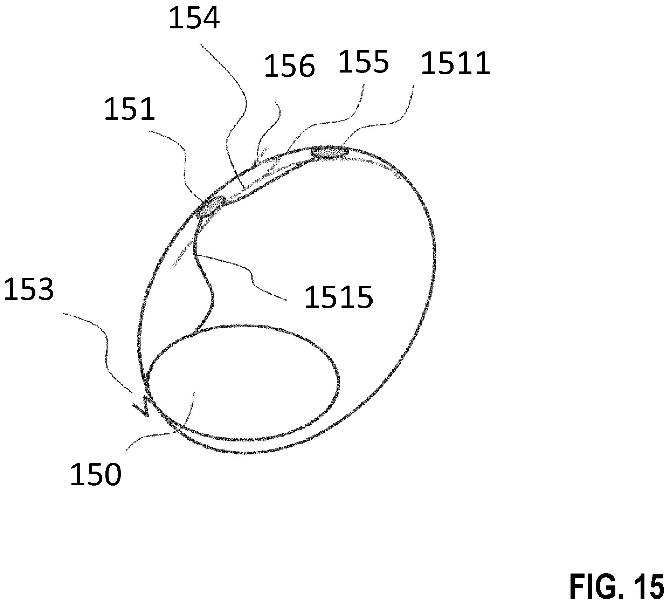

   - Figur 15: Realisierung des erfindungsgemäßen Stimulationssystems mit Selektionsmittel, das dazu ausgelegt ist, den Bereich des besagten Gewebes für die Stimulation zu wählen, wobei das Stimulationssystem über Träger verfügt

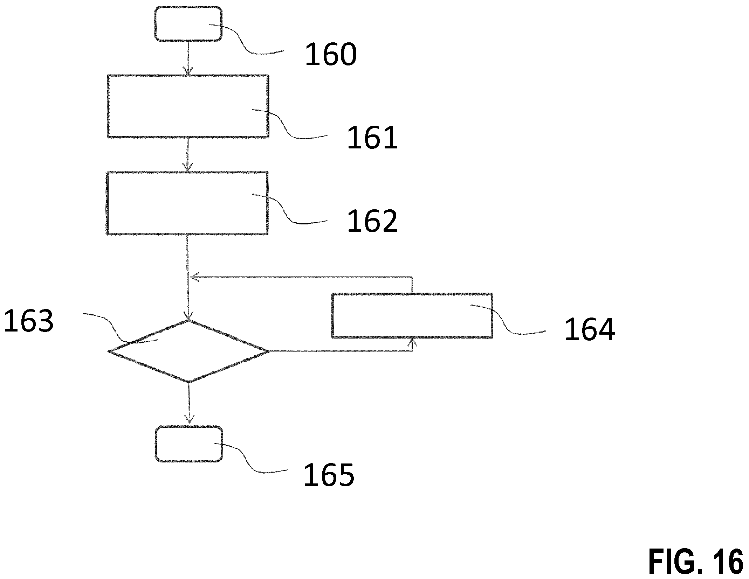

   - Figur 16: Ablaufplan der Methode für das erfindungsgemäße Stimulationssystem gemäß Ausführungsbeispielen

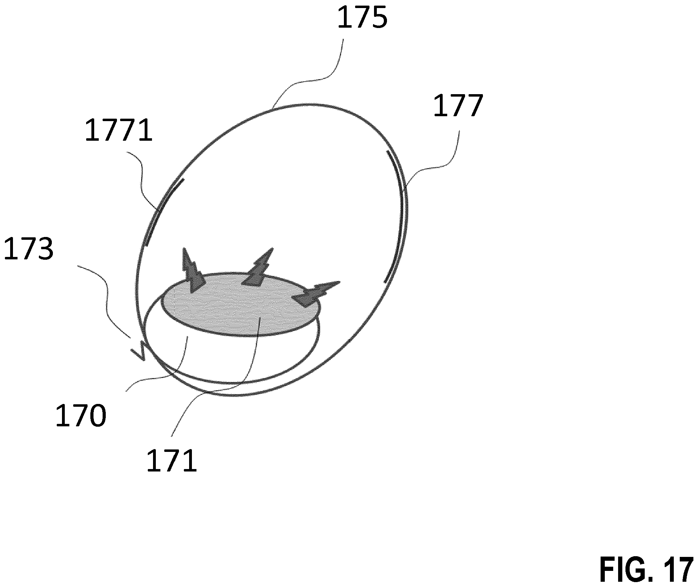

   - Figur 17: Schematische Darstellung der lokalen Vorbehandlung zur Erzeugung empfindsamer Bereiche für elektromagnetische Strahlung

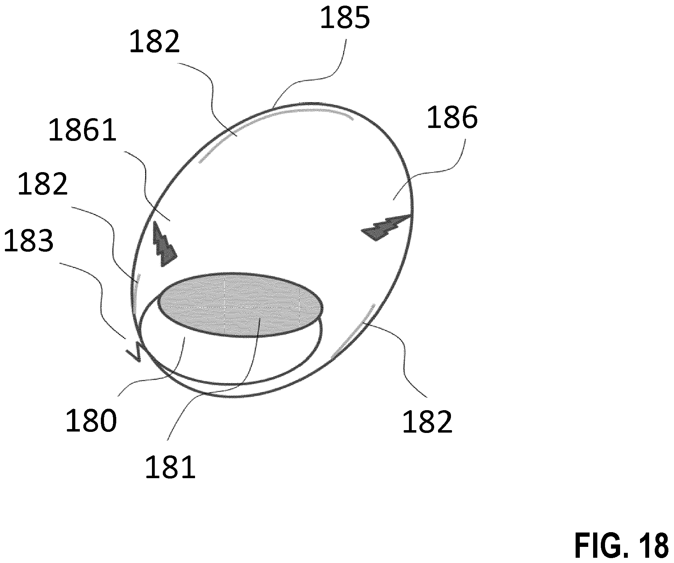

   - Figur 18: Schematische Darstellung der lokalen Vorbehandlung zur Erzeugung einer Maskierung ggf. empfindsamer Bereiche für elektromagnetische Strahlung.

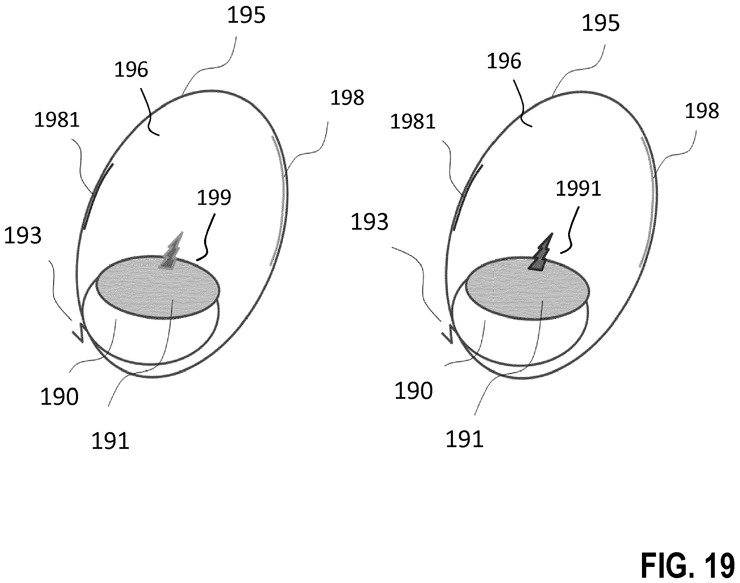

   - Figur 19: Beispiel mit lokal vorbehandelten Bereichen für die Stimulation mittels elektromagnetischer Strahlung

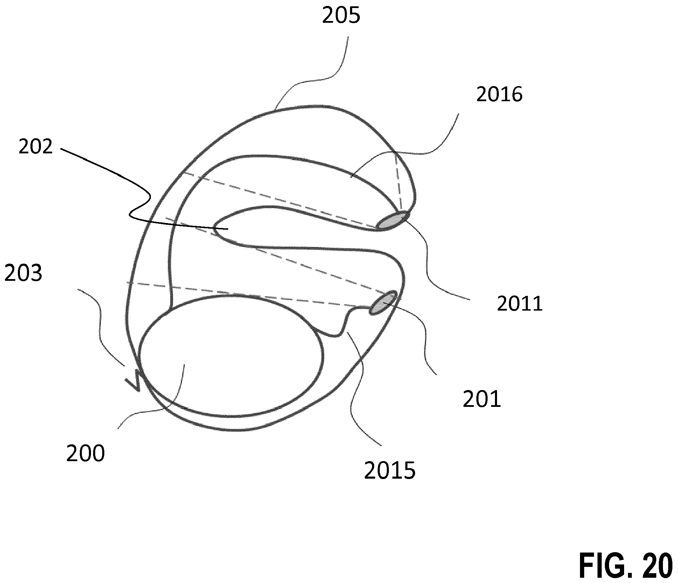

   - Figur 20: Schematische Darstellung der selektiven Therapie durch Platzierung der Aktuatoren, so dass anatomische Beschaffenheiten des Gewebes zur Abschattung genutzt werden, gemäß Ausführungsbeispielen der vorliegenden Erfindung.

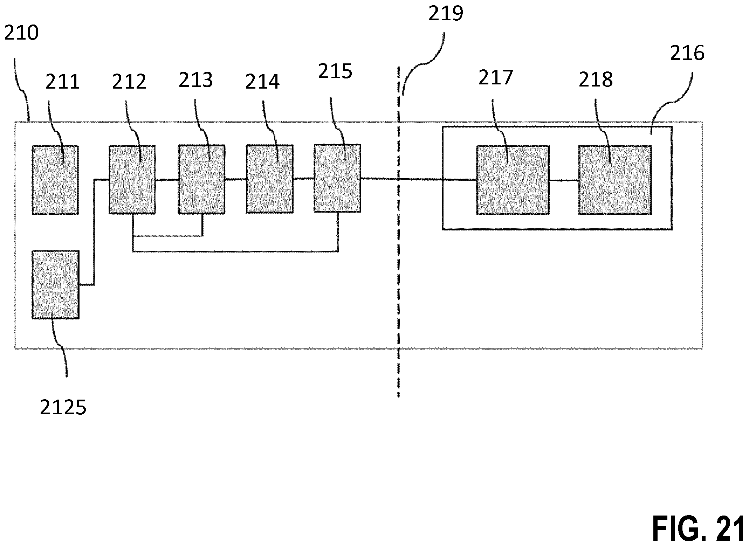

   - Figur 21: Blockschaltbild eines erfindungsgemäßen Stimulators gemäß einer Ausführungsform

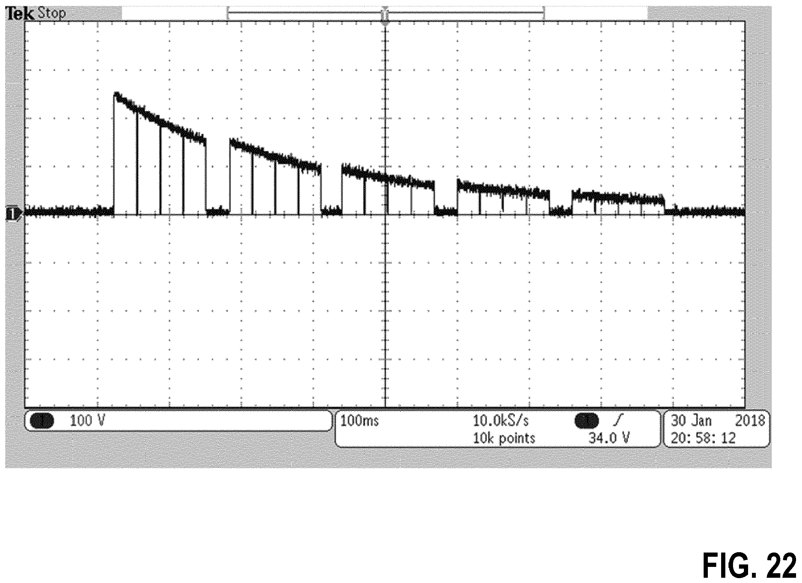

   - Figur 22: Schematische Darstellung der Pulsentladung eines ICDs, die einen erfindungsgemäßen Stimulator mit Therapiemöglichkeit mittels elektromagnetischer Strahlung zur Abgabe einer gepulsten Therapie ansteuert.

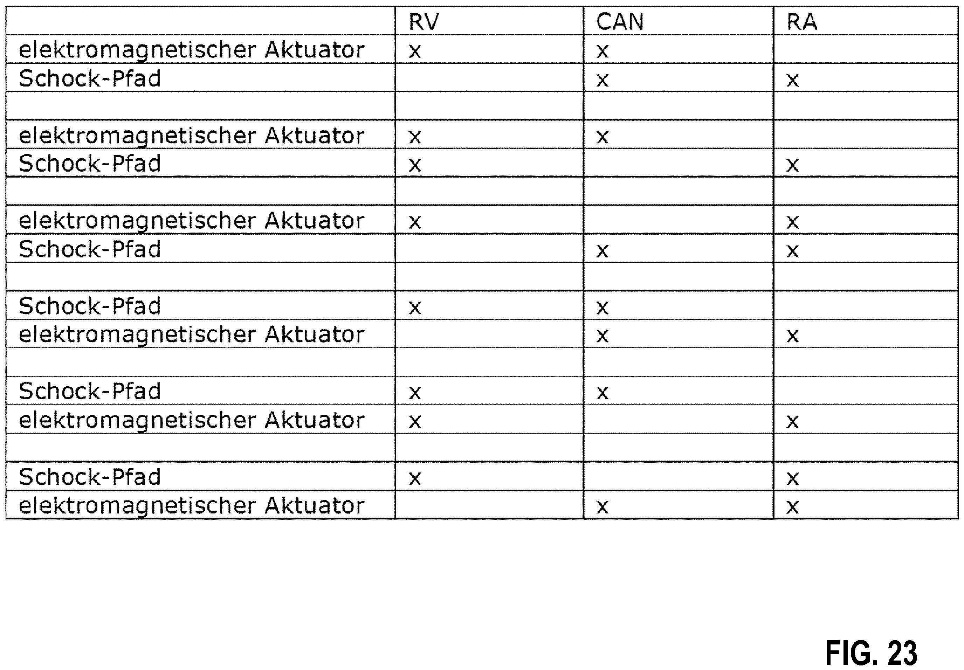

   - Figur 23: Tabelle, aufzeigend beispielhafte Kombinationen von Stimulations- und Schockvektoren zwischen rechtem Ventrikel, Gehäuse der Stimulationsvorrichtung und rechtem Atrium.

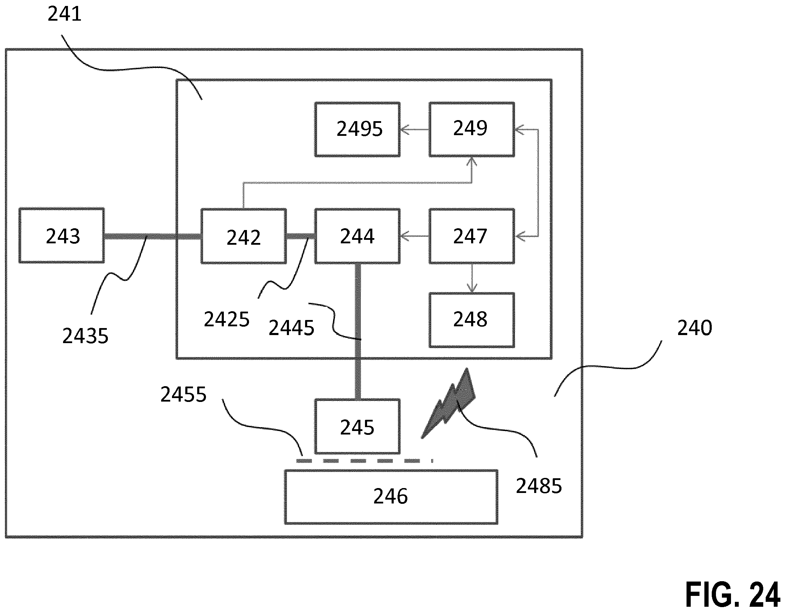

   - Figur 24: Schematische Darstellung der erfindungsgemäßen Vorrichtung gemäß einer Ausführungsform, bei der die Vorrichtung über ein umfassend ein Applikationsmittel zur Abgabe einer Substanz an Gewebe.

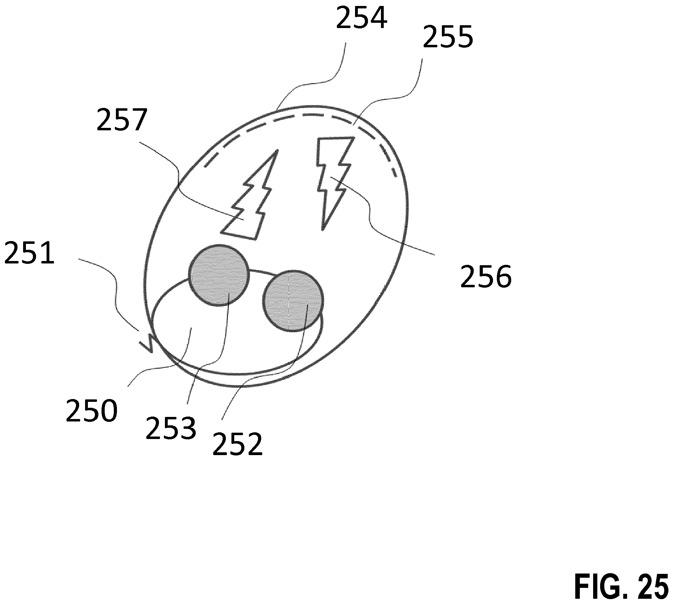

   - Figur 25: Schematische Darstellung der erfindungsgemäßen implantierbaren Vorrichtung nach einem Ausführungsbeispiel, wobei die Vorrichtung mit einer Fixiereinrichtung am Gewebe fixiert ist

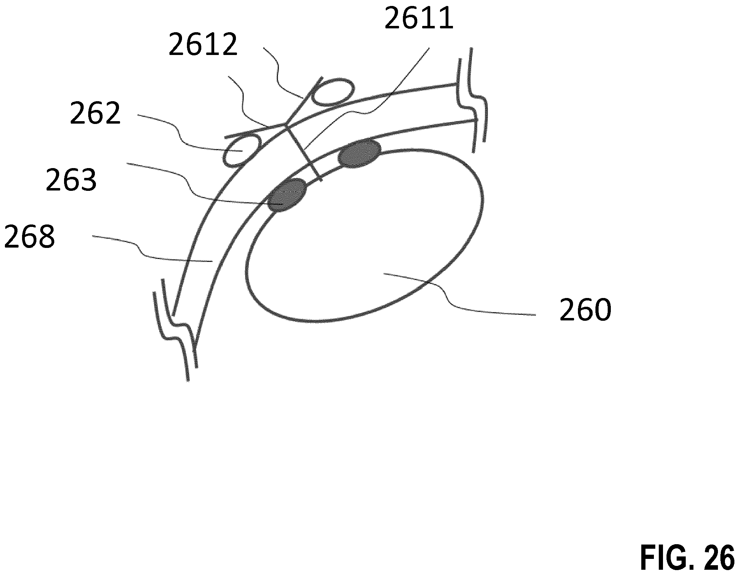

   - Fig. 26: Schematische Darstellung der erfindungsgemäßen Vorrichtung gemäß einem Realisierungsbeispiel. Die Vorrichtung verfügt über Aktuatoren bzw Sensoren für elektromagnetische Strahlung, die in der Fixiereinheit eingebaut sind.

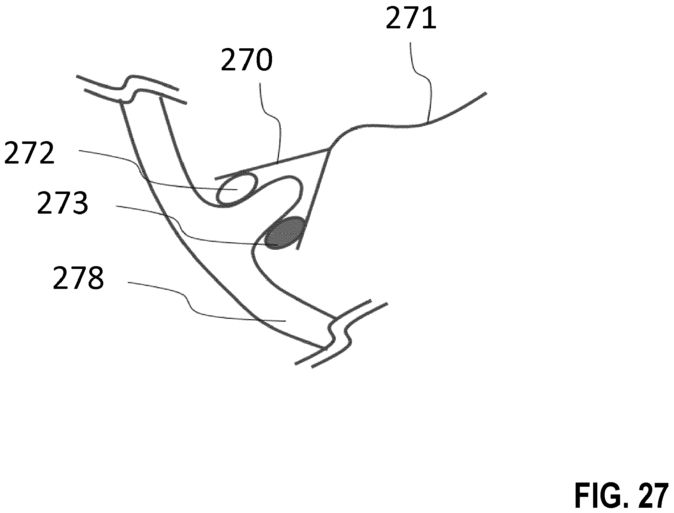

   - Fig. 27: Schematische Darstellung der erfindungsgemäßen Vorrichtung gemäß einem weiteren Realisierungsbeispiel. Die Vorrichtung verfügt über Aktuatoren bzw Sensoren für elektromagnetische Strahlung, die in der Fixiereinheit eingebaut sind.

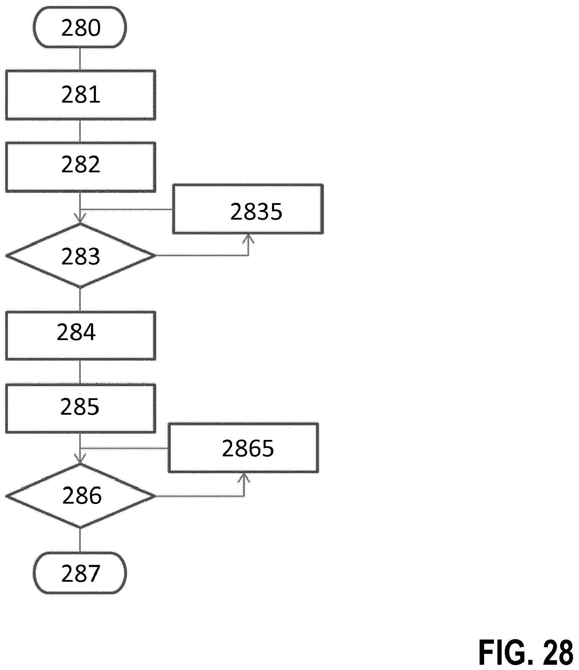

   - Figur 28: zeigt beispielhaft einen bevorzugten Signalverarbeitungsablauf der erfindungsgemäßen Methode

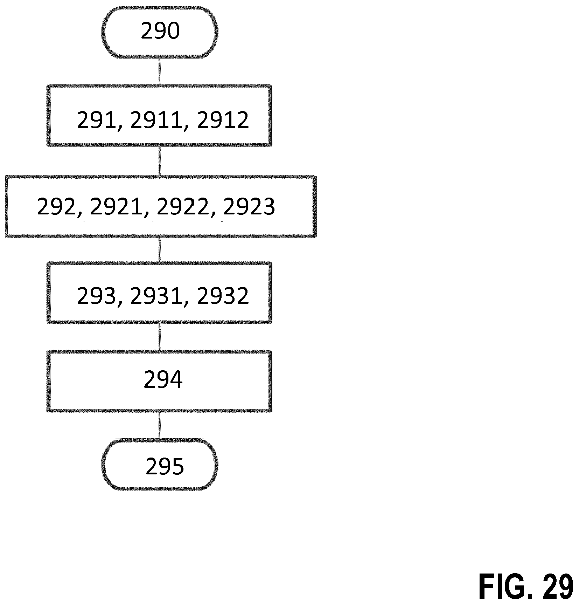

   - Figur 29: zeigt beispielhaft einen Signalverarbeitungsablauf der erfindungsgemäßen Methode

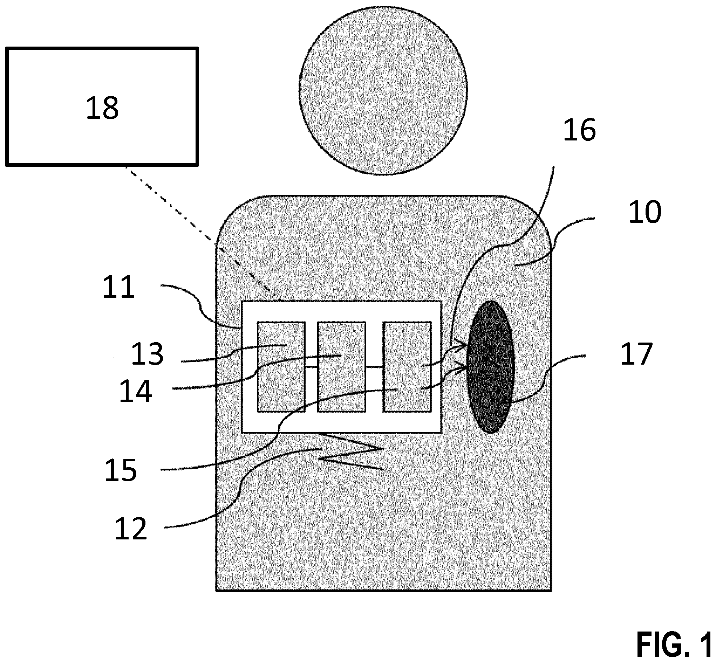

In Figur 1 ist ein erfindungsgemäßer implantierbarer Stimulator dargestellt. Dieser wird in das Körpergewebe des Patienten 10 implantiert und umfasst aus einem hermetisch versiegelten Gehäuse 11. Diese ist hier mit einer Fixiervorrichtung 12 im Körpergewebe verankert. Das Implantatgehäuse 11 beinhaltet eine Energiequelle 13, eine Steuereinheit 14 und einen Aktuator 15, der ausgebildet ist, eine elektromagnetische Strahlung 16 z.B. im Bereich des sichtbaren Lichtes an ein zuvor beispielsweise mittels genetischer, speziell optogenetischer Manipulation vorbehandeltes Körpergewebe abgeben zu können und beispielsweise ein Aktionspotential zu evozieren, d.h. eine Stimulation mittels elektromagnetsicher Strahlung auszulösen Zusätzlich kann das Implantat mittels eines externen Geräts 18, z.B. einer Telemetrieeinheit ausgelesen und/oder programmiert werden.

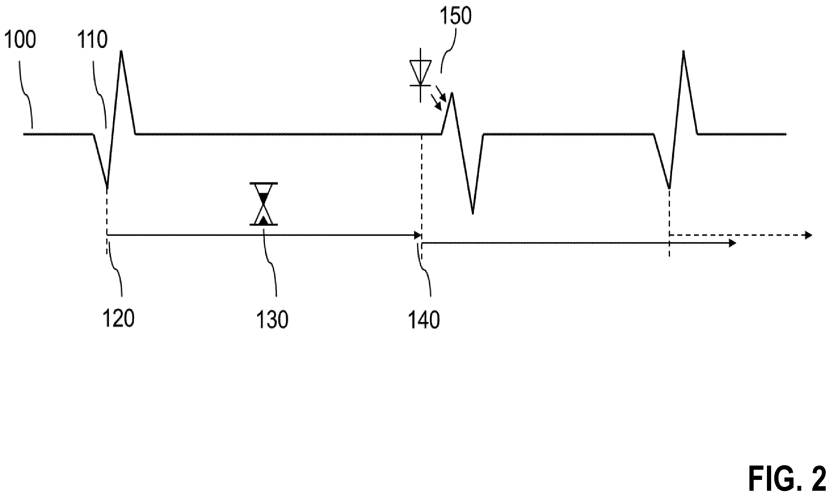

In der Figur 2 ist eine Methode zur Behandlung einer Bradykardie mittels Stimulation von genetisch manipuliertem Gewebe, bevorzugt optogenetisch manipuliertem Gewebe, illustriert. Im schematisierten EKG 100 ist zunächst eine reguläre Herzaktion 110 in Form eines intrinsischen QRS-Komplex dargestellt. Dieser wir bei dem vorgeschlagenen Verfahren zunächst registriert 120 und anschließend ein Zeitintervall 130 gestartet, dass einer erwarteten Herzrate entspricht. Läuft dieses Erwartungsintervall ab 140, ohne dass erneut ein intrinsischer QRS-Komplex registriert wird, so wird eine optische Stimulation 150 ausgelöst, die im vorzugsweise optogenetisch vorbehandelten Myokardgewebe einen stimulierten QRS-Komplex auslöst.

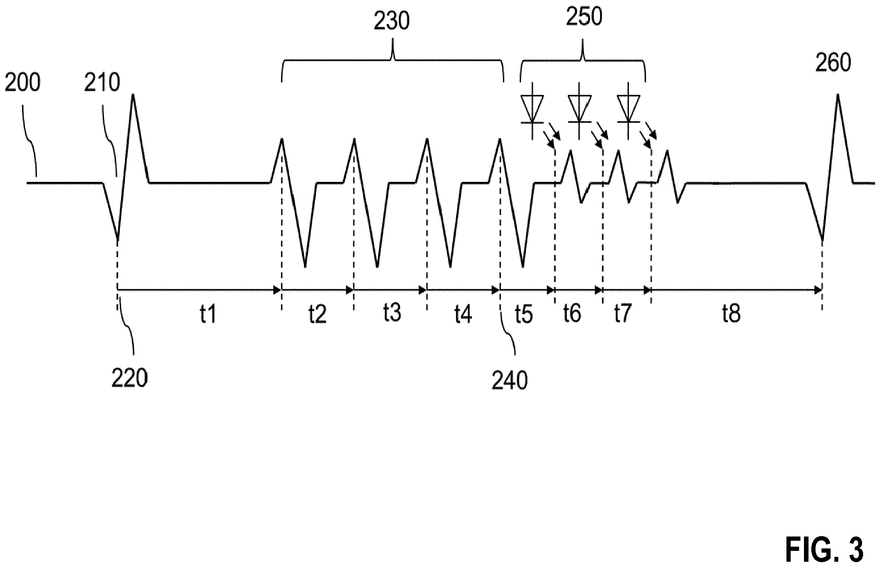

In der Figur 3 ist eine Methode zur Behandlung einer tachykarden Herzrhythmusstörung in Form einer ventrikulären oder atrialen Tachykardie illustriert. Im schematischen EKG 200 ist zunächst eine reguläre Kontraktion 210 einer Herzkammer dargestellt. Zur Überwachung des Herzrhythmus wir jede dieser Kontraktionen registriert und startet eine Zeitmessung bis zur nächsten registrierten Kontraktion (t1...t8). Im dargestellten Beispiel setzt nun eine zu schnelle Herzrate in Form einer Tachykardie 230 ein, die zunächst über einige Zyklen registriert und bestätigt wird 240. Nach Bestätigung 240 wird eine vorbestimmte optische Stimulationssequenz 250 an das vorzugsweise vorbehandelte Myokard abgegeben, welche eine Reihe von stimulierten Erregungen verursacht, die in der Regel die Tachykardie beenden und einen regulären Rhythmus wieder herstellen 260.

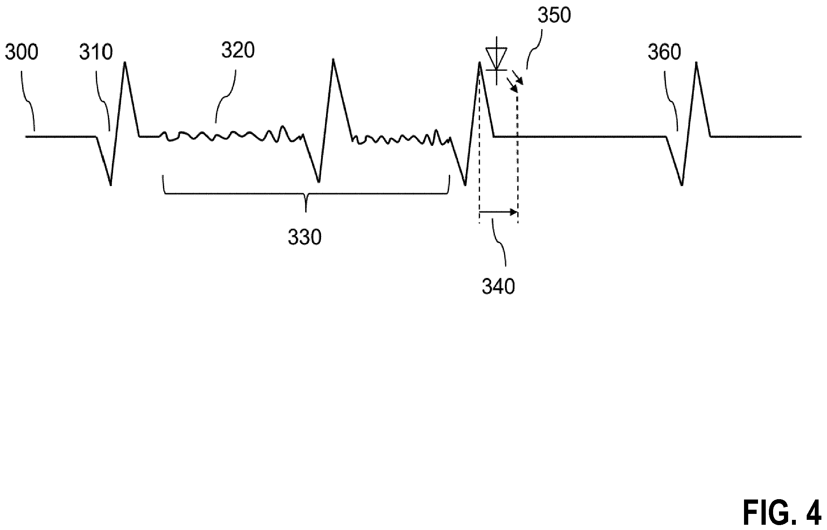

In der Figur 4 ist eine Methode zur Behandlung eines Vorhofflimmerns illustriert. Im schematischen EKG 300 ist zunächst eine QRS-Komplex in Sinusrhythmus 310 dargestellt. Anschließend setzt ein Vorhofflimmern 320 spontan ein. Dieses wird zunächst registriert und bestätigt 330. Mit der Bestätigung erfolgt die Abgabe einer optischen Stimulation 350 der Vorhöfe des vorbehandelten Herzens, jedoch synchronisiert und zeitversetzt zu einem QRS-Komplex (Kardioversion). Hiermit wird das Vorhofflimmern terminiert und ein regulärer Sinusrhythmus wird wieder hergestellt 360.

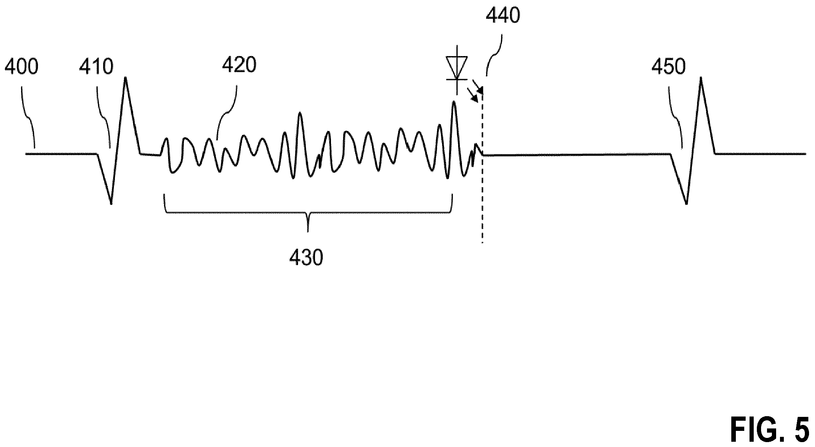

In der Figur 5 ist eine Methode zur Behandlung eines Kammerflimmerns illustriert. Im schematischen EKG 400 ist zunächst eine QRS-Komplex in Sinusrhythmus 410 dargestellt. Anschließend setzt spontan Kammerflimmern ein 420, welches zunächst registriert und anschließend bestätigt wird 430. Mit der Bestätigung wird ein großflächige optische Stimulation 440 im Bereich der vorzugsweise optogenetisch vorbehandelten Ventrikel ausgelöst, welche das Kammerflimmern terminiert und einen Sinusrhythmus 450 wiederherstellt.

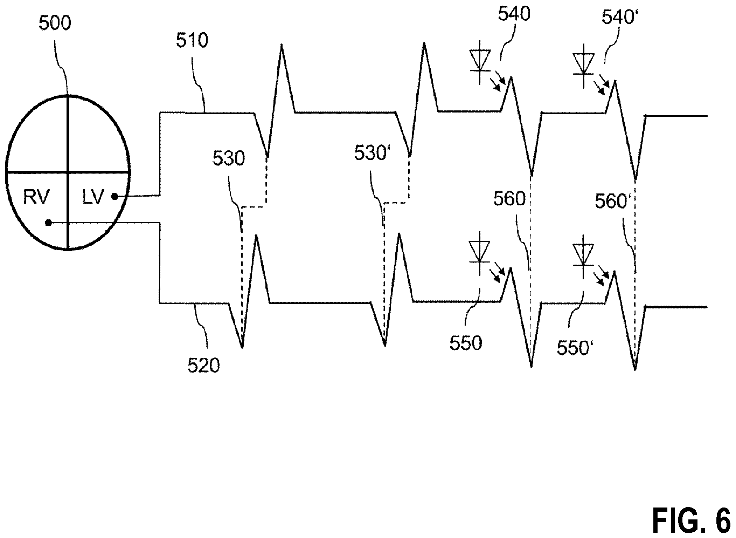

In der Figur 6 ist eine kardiale Resynchronisationsmethode illustriert zur Stimulation von genetisch manipuliertem Gewebe, bevorzugt optogenetisch manipuliertem Gewebe. Dargestellt sind schematisch die intrakardialen EKG-Ableitungen aus beiden Ventrikeln des Herzen 500: Linker Ventrikel (LV; 510) und rechter Ventrikel (RV, 520). Die EKG Ableitungen zeigen zunächst eine zeitliche Verzögerung 530 der linksventrikulären Herzaktionen gegenüber der rechtsventrikulären Herzaktionen. Dieser Versatz ist Ausdruck eines sogenannten Linksschenkelblocks, der bei fortgeschrittener struktureller Schädigung des Herzmuskels einer Therapie in Form einer Resynchronisation beider Ventrikel bedarf. Diese Resynchronisation erfolgt hier mittels simultaner oder quasisimultaner optischer Stimulation 540,550 beider Ventrikel des Herzens, so dass zeitgleich eine mechanische Kontraktion 560 beider Ventrikel dauerhaft hergestellt wird.

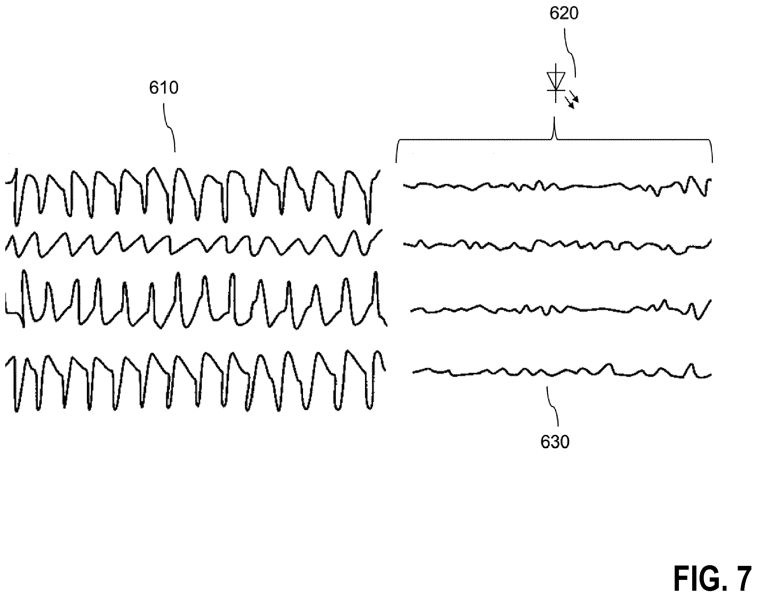

In der Figur 7 ist beispielhaft eine Neurostimulation von genetisch manipuliertem Gewebe, bevorzugt optogenetisch manipuliertem Gewebe, illustriert. Im gezeigten Beispiel ist auf der linken Seite ein EEG-Ausschnitt 610 eines epileptischen Anfalls abgebildet. Zu dessen Behandlung wird eine optische Stimulationssequenz 620 an eine oder mehrere vorzugsweise optogenetisch vorbehandelte Hirnregionen abgegeben, um eine Terminierung des epileptischen Anfalls herbeizuführen 630. Diese Darstellung soll die Methode der optischen Neurostimulation stellvertretend für alle weiteren Applikationen illustrieren, so z.B. Rückenmarksstimulation, weitere Applikationen der Deep Brain Stimulation oder kortikale Stimulation, isolierte Nervenstimulation, Muskelstimulation etc.

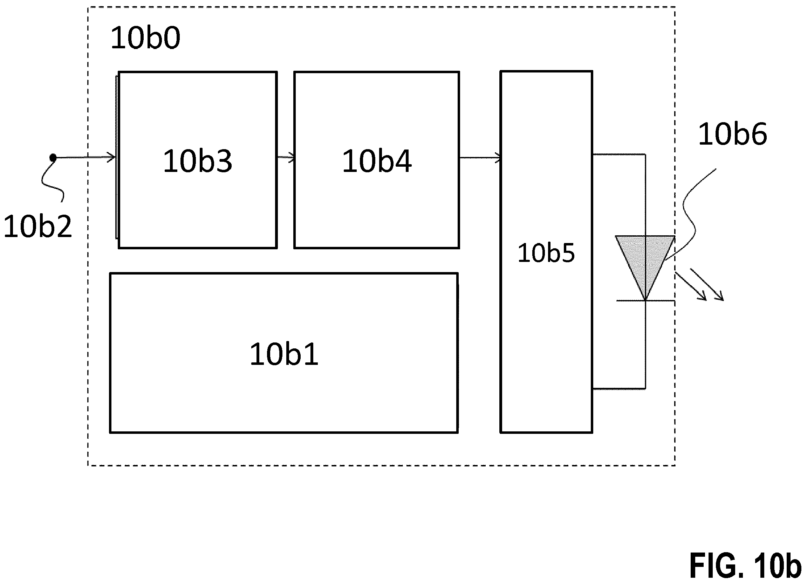

In der Figur 8 ist eine mögliche Ausführungsform eines erfindungsgemäßen implantierbaren Stimulators dargestellt. Dieser besteht aus einer Batterie 81 und einer Implantatselektronik 82, die die Komponenten des Blockschaltbildes aus Figur 10 beinhaltet, beides in einem hermetischen Gehäuse untergebracht. An der Unterseite des Gehäuses ist eine Lichtquelle 83 angebracht, die ebenfalls hermetisch versiegelt ist, jedoch so, dass das Frequenzspektrum dieser Lichtquelle die hermetische Versiegelung durchdringen und das Zielgewebe -hier Myokard- 85 erregen kann. Die Fixierung des Implantates erfolgt hier mit einer Helix 84 im Myokard 85.

In der Figur 9 ist der implantierbare Stimulator aus Figur 8 bestehend aus einer Batterie 81 und einer Implantatselektronik 82 dargestellt, jedoch mit einer alternativen Fixiervorrichtung 94, 94' in Form von seitlich angebrachten Widerhaken aus z.B. Nitinol. An der Unterseite des Gehäuses ist eine Lichtquelle 93 angebracht, die hermetisch versiegelt ist, derart, dass das Frequenzspektrum dieser Lichtquelle die hermetische Versiegelung durchdringen und das Zielgewebe -hier Myokard- 95 erregen kann.

In der Figur 10 ist das Blockschaltbild des implantierbaren Stimulators 100 dargestellt. Dieses Umfasst eine Energiequelle 101, ein Sensorinterface 102 zur Wahrnehmung eines eine Herzaktion repräsentierenden Merkmals, verbunden mit einer Wahrnehmungseinheit 103, die wiederum die Wahrnehmung von Herzaktionen an die angeschlossene Steuereinheit 104 signalisiert. Diese Steuereinheit 104 ist mit einem Therapiegenerator 105 verbunden, welcher bei Erhalt eines Triggersignals von der Steuereinheit 104 eine an den Therapiegenerator angeschlossene LED 106 anregt und damit ein das Myokardgewebe stimulierendes Lichtsignal aussendet. Der Therapiegenerator 105 kann dabei das Lichtsignal in seiner Intensität, Dauer, Signalform und Farbe variieren.

In der Figur 8a ist eine mögliche Ausführungsform eines erfindungsgemäßen implantierbaren Stimulators mit Therapieerfolgskontrolle dargestellt. Dieser besteht aus einer Batterie 8a1 und einer Implantats-Elektronik 8a2, die die Komponenten des Blockschaltbildes aus Figur 9a beinhaltet, beides in einem hermetischen Gehäuse untergebracht. An der Unterseite des Gehäuses ist eine Lichtquelle 8a3 angebracht, die ebenfalls hermetisch versiegelt ist, jedoch so, dass das Frequenzspektrum dieser Lichtquelle die hermetische Versiegelung durchdringen und das Zielgewebe -hier Myokard- 8a5 erregen kann. Die Fixierung des Implantates erfolgt hier mit einer Helix 8a4 im Myokard 8a5. In diesem Ausführungsbeispiel umfasst die Implantats-Elektronik 8a2 zusätzlich ein 3D-Beschleunigungssensor 8a6, der zur Therapieerfolgskontrolle derart eingesetzt wird, dass nach jeder optischen Stimulation ausgewertet wird, ob eine Beschleunigung des am Myokard fixierten Implantates festgestellt wird. In diesem Falle gilt die Stimulation als effektiv, da eine Kontraktion des Herzgewebes zur Beschleunigung führt. Bleibt die Beschleunigung aus, gilt die Stimulation als ineffektiv und wird z.B. mit höherer Amplitude wiederholt oder alternativ eine ineffektive Stimulation an ein Fernüberwachungssystem signalisiert.

In der Figur 9a ist das Blockschaltbild des implantierbaren Stimulators 9a0 dargestellt. Dieses Umfasst eine Energiequelle 9a1, ein Sensorinterface 9a2 zur Wahrnehmung eines eine Herzaktion repräsentierenden Merkmals, verbunden mit einer Wahrnehmungseinheit 9a3, die wiederum die Wahrnehmung von Herzaktionen an die angeschlossene Steuereinheit 9a4 signalisiert. Diese Steuereinheit 9a4 ist mit einem Therapiegenerator 9a5 verbunden, welcher bei Erhalt eines Triggersignals von der Steuereinheit 9a4 eine an den Therapiegenerator angeschlossene LED 9a6 anregt und damit ein das Myokardgewebe stimulierendes Lichtsignal aussendet. Der Therapiegenerator 9a5 kann dabei das Lichtsignal in seiner Intensität, Dauer, Signalform und Farbe variieren.
Die Wahrnehmungseinheit 9a3 ist ferner mit ein 3D-Beschleunigungssensor 9a7 verbunden, der zur Therapieerfolgskontrolle derart eingesetzt wird, dass nach jeder optischen Stimulation ausgewertet wird, ob eine Beschleunigung des am Myokard fixierten Implantates festgestellt wird. In diesem Falle gilt die Stimulation als effektiv, da eine Kontraktion des Herzgewebes zur Beschleunigung führt. Bleibt die Beschleunigung aus, gilt die Stimulation als ineffektiv und wird z.B. mit höherer Intensität, Dauer, alternativer Signalform oder anderer Farbe wiederholt.

Figur 10a zeigt beispielhaft zweidimensionale Charakteristiken elektromagnetischer Strahlers mit räumlich selektiver Wirkung.

In der Figur 8b ist eine mögliche Ausführungsform eines erfindungsgemäßen implantierbaren Defibrillators dargestellt. Dieser besteht aus einer Hochleistungs-LED 8b1 und einer verkapselten Implantatselektronik nebst Energiequelle 8b2, die die Komponenten des Blockschaltbildes aus Figur 3 beinhaltet, beides in einem hermetischen Gehäuse untergebracht. An der Unterseite des Gehäuses ist eine weitere lokale Lichtquelle 8b3 angebracht, die derart angebracht und dimensioniert ist, dass diese nur einen lokale Depolarisation im vorbehandelten Myokard 8b5 auszulösen vermag. Die Fixierung des Implantates erfolgt hier mit einer Helix 8b4 im Myokard 8b5. Die Hochleistungs-LED 8b1 ist derart dimensioniert, das diese zum Zwecke der Defibrillation nahezu das ganze Herz "durchscheinen" kann, so dass eine gleichzeitige Depolarisation aller erregbaren Myokardzellen im Augenblick der Defibrillation möglich ist.

In der Figur 9b ist der implantierbare Stimulator aus Figur 8b dargestellt, jedoch mit einer alternativen Fixiervorrichtung 9b4, 9b4' in Form von seitlich angebrachten Widerhaken aus Nitinol. An der Unterseite des Gehäuses ist eine Lichtquelle 9b3 angebracht, die hermetisch versiegelt ist, derart, dass das Frequenzspektrum dieser Lichtquelle die hermetische Versiegelung durchdringen und das Zielgewebe -hier Myokard- 9b5 erregen kann.

In der Figur 10b ist das Blockschaltbild des implantierbaren Stimulators lObO gemäß Figur 8b dargestellt. Dieses Umfasst eine Energiequelle 10b1, ein Sensorinterface 10b2 zur Wahrnehmung eines eine Herzaktion repräsentierenden Merkmals, verbunden mit einer Wahrnehmungseinheit 10b3, die wiederum die Wahrnehmung von Herzaktionen an die angeschlossene Steuereinheit 10b4 signalisiert. Diese Steuereinheit 10b4 ist mit einem Therapiegenerator 10b5 verbunden, welcher bei Erhalt eines Triggersignals von der Steuereinheit 10b4 eine an den Therapiegenerator angeschlossene LED 10b6 anregt und damit ein das Myokardgewebe stimulierendes Lichtsignal aussendet. Der Therapiegenerator 10b5 kann dabei das Lichtsignal in seiner Intensität, Dauer, Signalform und Farbe variieren.

In der Figur 11 wird eine typische Realisierung dargestellt. Das Implantat 110 ist mit einer Fixiereinrichtung 113 an Organgewebe befestigt und verfügt über einen elektromagnetischen Strahler 111 der mittels der Maske 112 maskiert ist, um nur innerhalb der Wirkkegel 114 und 1141 elektromagnetische Energie auf das zu therapierende Gewebe 115 einstrahlen zu lassen.

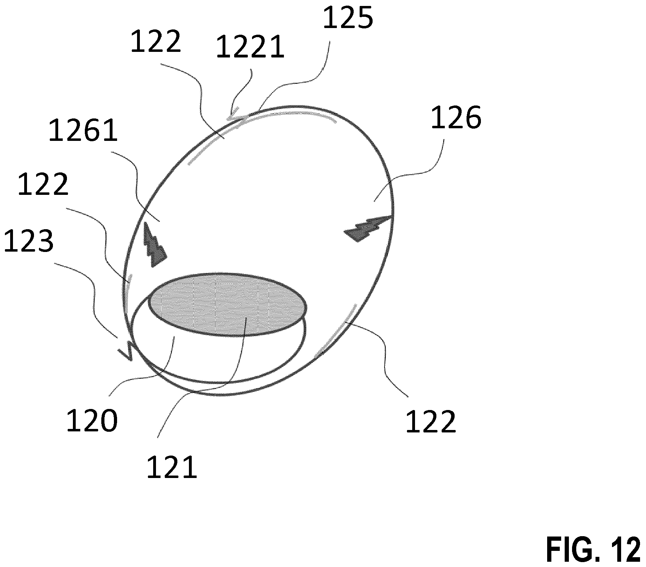

In der Figur 12 ist die Realisierung offenbart, bei der die Maske 122 eine zweite Einheit darstellt und unabhängig vom Implantat mit einer Fixiervorrichtung 1221 befestigt ist, um zu verhindern dass elektromagnetische Energie in die abgedeckten Geweberegionen eindringt. Das Implantat 120 ist mit einer Fixiereinrichtung 123 an Organgewebe befestigt und verfügt über einen elektromagnetischen Strahler 121 der mittels der Maske 122 maskiert ist.. Die Therapie wirkt nur in den nichtmaskierten Regionen 126 und 1261 auf das zu therapierende Gewebe 125.

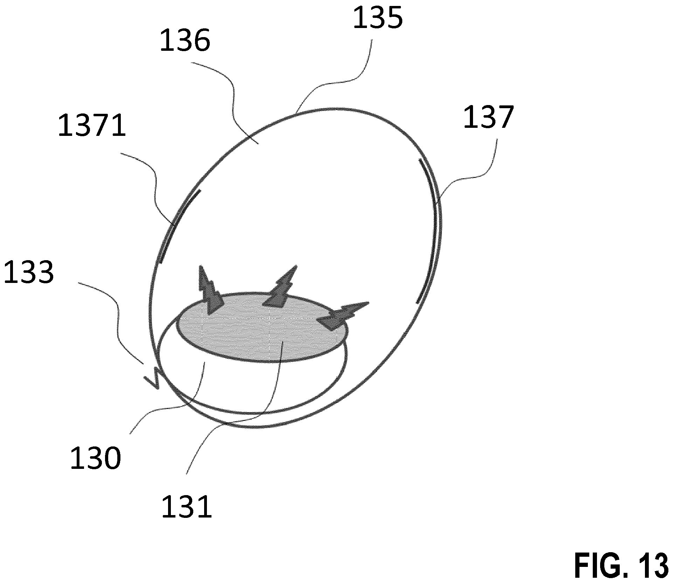

Figur 13 zeigt eine Realisierung mit lokal vorbehandeltem Gewebe. Der Wirkbereich 136 der Strahlung erreicht zwar das gesamte Gewebe 135, wirkt jedoch nur den in den Regionen 137 und 1371.. Das Implantat 130 ist mit einer Fixiereinrichtung 133 an Organgewebe befestigt und verfügt über einen elektromagnetischen Strahler 131. Die Therapie wirkt nur in den vorbehandelten Regionen 137 und 1371 auf das zu therapierende Gewebe 135.

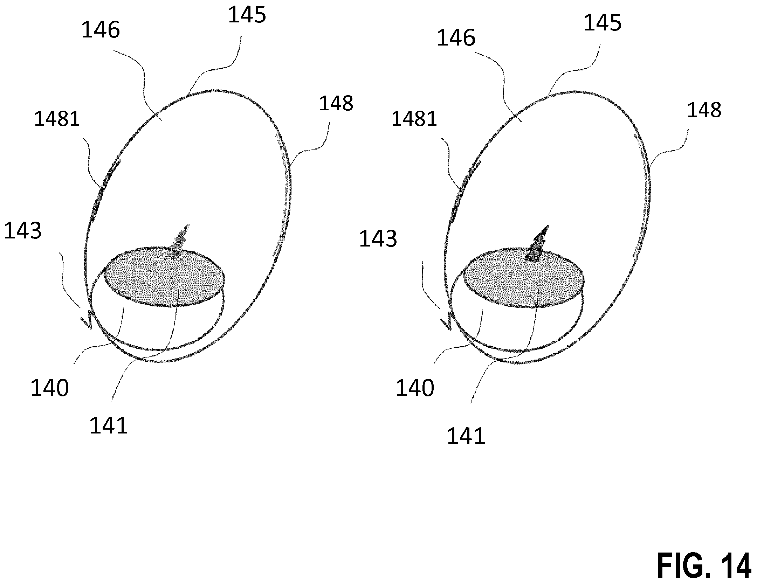

Figur 14 offenbart eine Lösung mit lokal frequenzspezifisch (oder polarisationsspezifisch) reagierendem oder dahingehend vorbehandeltem Gewebe. Je nach Frequenz(band) bzw. Polarisation des Strahlers zeigt nur die eine 148 oder die andere 1481 Region Wirkung. Der Wirkbereich 146 der Strahlung erreicht zwar das gesamte Gewebe 145, wirkt jedoch nur den in den Regionen 148 und 1481. Das Implantat 140 ist mit einer Fixiereinrichtung 143 an Organgewebe befestigt und verfügt über einen elektromagnetischen Strahler 141.

Figur 15 zeigt eine Lösung mit distal zum Implantatgehäuse liegenden Strahlern 151 und 1511 die entweder direkt (nicht gezeigt) oder mittels eines Trägers 154 mit Fixierung 156 an des zu therapierende Gewebe angebracht sind. Dabei übernimmt der Träger auch die Rolle einer Maske und schattet das restliche Organ ab. Die Strahler werden vom Implantat 150 über eine Leitung 1515 versorgt. Alternativ kann das Implantat selbst die Rolle eines Trägers übernehmen. Die Strahler sind dann direkt am Gehäuse befestigt. Das Implantat 150 ist mit einer Fixiereinrichtung 153 an Organgewebe befestigt.

In der Figur 16 ist der Ablaufplan der Methode für das erfindungsgemäße Stimulationssystem gemäß Ausführungsbeispielen gezeigt. Nach Start 160 erfolgt die Vorbehandlung 161 gefolgt von der Implantation und Befestigung des Stimulators 162. Danach wird der Test 163 der Therapiewirksamkeit durchgeführt. Ist dieser nicht erfolgreich wird im Vorgang 164 nachgebessert. Anderen falls ist die Methode beendet 165.

Figur 17 zeigt das Gewebe175 mit den Vorbehandelten Bereichen 177 und 1771. Der Stimulator 170 mit elektromagnetischem Aktuator 171 wird mit der Fixierung 173 am Gewebe befestigt.

Figur 18 zeigt das Gewebe185 mit den durch Vorbehandlung maskierten Bereichenl82. Es sind somit nur die Bereiche 186 und 1861 für die vom Aktuator 181 abgegebenen Strahlung stimulierbar. Das Implantat 180 ist mit einer Fixiereinrichtung 183 an Organgewebe befestigt und verfügt über einen elektromagnetischen Strahler 181.

Figur 19 zeigt ein Beispiel mit lokal vorbehandelten Bereichen 198 und 1981 die jedoch für unterschiedlichen Frequenzen der elektromagnetischen Strahlung empfindsam sind. Die selektive Wirkung der Therapie kommt dadurch zustande, dass der Aktuator 191 elektromagnetische Strahlung einmal auf der einen Frequenz 199 und ein anderes Mal auf einer anderen Frequenz 1991 abgibt. Der Wirkbereich 196 der Strahlung erreicht zwar das gesamte Gewebe 195, wirkt jedoch nur den in den Regionen 198 und 1981. Das Implantat 190 ist mit einer Fixiereinrichtung 193 an Organgewebe befestigt und verfügt über einen elektromagnetischen Strahler 191.

Figur 20 zeigt ein Beispiel für eine selektive Therapie durch Platzierung der Aktuatoren so dass anatomische Beschaffenheiten des Gewebes zur Abschattung genutzt werden. Figur 20 zeigt wie selektive Therapie erreicht wird indem durch Platzierung der Aktuatoren 201 und 2011 (mit dem Stimulator 200 verbunden durch Leitungen 2015 und 2016) anatomische Formen 202 des Gewebes 205 zur Abschattung genutzt werden. Das Implantat 200 ist mit einer Fixiereinrichtung 203 an Organgewebe befestigt.

In der Figur 21 ist das Blockschaltbild des in den Ansprüchen beschriebenen Stimulators mit optischem Therapieausgang dargestellt. Der Stimulator 210 umfasst eine Energiequelle 211, mindestens einen Energiespeicher 214, eine Vorrichtung zur Ladung 213 des Energiespeichers 214, eine Vorrichtung zur Freigabe der Energie 215. Des Weiteren ist dargestellt ein Aktuator 216, wobei der Aktuator über eine Lichtquelle 218, sowie einen Strombegrenzer 217 (z.B. Widerstand oder Diode) verfügt. Der Stimulator 210 verfügt über eine Steuereinheit 212. Stimulator 210 umfasst eine Wahrnehmungseinheit 2125. Aktuator 216 kann als eine von der übrigen erfindungsgemäßen Vorrichtung trennbare Einheit realisiert sein und über eine Schnittstelle 219 zur Kontaktierung verfügen.

In der Figur 22 ist die Pulsentladung eines ICD dargestellt, die einen erfindungsgemäß realisierten optischen Therapieaktuator zur Abgabe einer gepulsten Therapie ansteuert.

In einer erfindungsgemäßen Ausführungsform können bereits bekannte elektrische Stimulationsvorrichtungen (z.B. Herzschrittmacher, Neuro-Stimulator) mit einem Aktuator für die Aussendung von elektromagnetischer Strahlung ergänzt werden, um eine Stimulation mittels elektromagnetischer Wellen durchzuführen. Abgesehen vom Aktuator bedarf es dabei für das Stimulationsgerät nur geringfügiger bis keinerlei Modifikationen.

Figur 23 zeigt tabellarisch beispielhafte Kombinationen von Stimulations- und Schockvektoren zwischen rechtem Ventrikel RV, Gehäuse der Stimulationsvorrichtung CAN und rechtem Atrium RA. Betrachtet man exemplarisch die zweite und dritte Zeile der Tabelle, so stellt diese eine Kombination zweier Vektoren dar: ein erster Vektor von Gehäuse CAN zum rechten Ventrikel RV für die Stimulation durch einen Aktuator gemäß der Erfindung, sowie einen zweiten Schockvektor zwischen Gehäuse CAN und rechtem Atrium RA. Bei einem handelsüblichen ICD können diese elektrischen Pole realisiert sein durch eine rechtsventrikuläre Schockwendel, das ICD-Gehäuse und eine supraventrikuläre Schockwendel. Ein Vektor führt jeweils von einem ersten durch das Kreuz gekennzeichneten Pol zu einem zweiten. Die Stimulation kann durch elektromagnetische Strahlung über einen Aktuator gemäß der Erfindung durchgeführt werden, oder durch bekannte elektrische Stimulation, oder durch eine Kombination der beiden.

Figur 24 zeigt schematisch eine Ausführungsform der erfindungsgemäßen Vorrichtung umfassend ein Applikationsmittel zur Abgabe einer Substanz an Gewebe. Das Gerät 241 ist im Köper 240 implantiert. Es verfügt über ein Reservoir 242 für die therapeutische Substanz und Zuleitungen 2435, 2425 und 2445. Das Reservoir wird über einen Port 243 befüllt. Die Pumpe pumpt gesteuert von der Steuereinheit 247 die Substanz zur Gerät-Gewebe-Schnittstelle oder Applikationsmittel 245, die über eine optionale Maske 2455 das Organ 246 mit dieser Substanz behandelt. Eine Kontrolleinheit 249 nimmt u.a. den Reservoirstand wahr und meldet diesen über die Telemetrieeinheit 2495 nach außen. Zum Test der Wirksamkeit gibt von 247 gesteuert die Therapieeinheit 248 ein Testsignal 2485 ab. Die Reaktion wird über eine Wahrnehmungseinheit (hier nicht gezeigt) festgestellt und der Kontrolleinheit gemeldet.

Figur 25 zeigt die erfindungsgemäße implantierbare Vorrichtung 250 die mit Fixiereinrichtung 251 am Gewebe 254 fixiert ist und über einen Sensor 252 zur Wahrnehmung von elektromagnetischer Strahlung 256 verfügt, die als primäre Strahlung von den zu beobachtenden (optional vorbehandelten) erregbaren Zellstrukturen 255 ausgeht, wenn diese Aktionspotentiale ausbilden.
In einer alternativen Realisierung/Anwendungsszenario verfügt die implantierbare Vorrichtung 250 über einen zusätzlichen Aktuator 253 für die Erzeugung von elektromagnetischer Strahlung 257. Diese wird von den zu beobachtenden (optional vorbehandelten) erregbaren Zellstrukturen 255 in Abhängigkeit ihrer Aktionspotentiale moduliert und kehrt als sekundäre Strahlung 256 zum Sensor 252 zurück.

In der Figur 26 wird eine Realisierung mit in der Fixiereinheit eingebauten Aktuatoren und Sensoren für elektromagnetische Strahlung offenbart. Die Fixiereinheit 261 besteht hier aus einem Schaft 2611 mit klappbaren, arretierbaren Armen 2612 die z.B. die Sensoren 262 tragen. Die Aktuatoren 263 sind gegenüberliegend z.B. am Gehäuse 260 fixiert. Bei Implantation werden die Arme in Richtung des Schaftes ausgerichtet, durch die Organwand 268 gestochen und dann umgeklappt und in der Stellung arretiert, dass sie den Aktuatoren jenseits der Wand passend gegenüberliegen

In der Figur 27 wird eine weitere Realisierung mit in der Fixiereinheit eingebauten Aktuatoren 273 und Sensoren 272 für elektromagnetische Strahlung offenbart. Dabei ist die Fixiereinheit 271 der Aktoren 273 und Sensoren 272 als Zwacke 270 ausgeführt. Eine Zuleitung 271 führt zum Gerät (nicht gezeigt).
Figur 28 zeigt beispielhaft einen bevorzugten Implantationsablauf der erfindungsgemäßen Methode. Die dargestellten Schritte sind:
- 280 Start der Implantation
- 281 Vorbehandlung des Zielgewebes
- 282 Feststellung des geeigneten Implantationsortes
- 283 Positionierung der Vorrichtung, solange bis der geeignete Implantationsort erreicht ist
- 2835 Nachstellen der Position
- 284 Fixierung
- 285 Parametereinstellung und Start der Wahrnehmungsmethode (inkl. Aufbereitung und Analyse)
- 286 Testung
- 2865 Nachstellen der Parameter
- 287 Ende der Implantation.

Figur 29 zeigt beispielhaft einen bevorzugten Signalverarbeitungsablauf der erfindungsgemäßen Methode. Die dargestellten Schritte sind:
- 290 Start der Wahrnehmung (Messung)
- 291 Verstärkung,
- 2911 Demodulation,
- 2912 analoge Filterung,
- 292 AD-Wandlung,
- 2921 digitale Filterung,
- 2922 Bestimmung der Signalleistung,
- 2923 Schwellwertbestimmung,
- 293 Segmentierung,
- 2931 Ereigniserkennung,
- 2932 Bestimmung von Periodizität,
- 294 Klassifikation von Rhythmen
- 295 Ende der Wahrnehmung (Messung)

Die Erfindung eliminiert ganz oder teilweise die nachteiligen Wirkungen galvanisch eingekoppelter elektrischer Therapieströme für die Therapie von Herzgewebe, neuronalem Gewebe oder Muskelgewebe.
Dieser selektive Therapieansatz eröffnet neue Möglichkeiten für die multifokale Therapie ohne für jeden Stimulationsort eine gesonderte Sonde implantieren zu müssen. Durch die großflächig mögliche multifokale Therapie können Erregungsmuster angeregt werden, die die natürlichen spatio-temporalen Verhältnisse viel besser als bisher abbilden.
Die Anforderungen an den Energiebedarf von derartigen Implantaten kann erheblich gesenkt werden. Es werden ferner völlig neue Bauformen derartiger Implantate möglich.

Im Kontext der Erfindung werden folgende Begriffe als Synonym für die erfindungsgemäße implantierbare Vorrichtung zur Wahrnehmung von elektromagnetischen Wellen, die von genetisch manipuliertem Gewebe ausgesendet werden, und/oder zur Stimulation von genetisch manipuliertem Gewebe mittels elektromagnetischer Wellen verwendet: Stimulator, Stimulationsgerät, Stimulationsvorrichtung, Vorrichtung/Gerät zur Stimulation, Stimulationssystem (Vorrichtung ist zumindest Teil eines solchen beschriebenen Stimulationssystems).

Im Kontext der Erfindung:
- wird unter "Wellenzug" eine durchgehende elektromagnetische Welle verstanden;
- werden die Begriffe "Elektromagnetische Strahlung", "Elektromagnetische Welle" als Synonym verwendet
- ATP hat die Bedeutung "Anti tachycardia pacing", IPG hat die Bedeutung "Implantable Pulse Generator", ICD hat die Bedeutung "Implantable Cardioverter-Defibrillator"

## Patentansprüche

1. Vorrichtung, mindestens umfassend:
- eine Energiequelle,
- einen Energiespeicher,
- eine Elektronikeinheit,
wobei die Vorrichtung zur Implantation im menschlichen oder tierischen Körper ausgelegt ist,
- einen Aktuator, der mit dem Energiespeicher gekoppelt ist und dazu ausgelegt ist, durch Entladung des Energiespeichers elektromagnetische Wellen auszusenden.

2. Vorrichtung nach Anspruch 1, wobei der Aktuator dazu ausgelegt ist, durch das Aussenden elektromagnetischer Wellen im Frequenzbereich 10¹³-10²⁰ Hz die Wahrnehmung und/oder Evozierung von Aktionspotentialen in genetisch manipuliertem Gewebe hervorzurufen.

3. Vorrichtung nach mindestens einem der vorigen Ansprüche, wobei der Energiespeicher einen Kondensator und/ oder eine Spule aufweist.

4. Vorrichtung nach mindestens einem der vorigen Ansprüche, wobei die Elektronikeinheit eine Steuereinheit umfasst, wobei die Steuereinheit mindestens eine der folgenden Eigenschaften aufweist:
- sie ist dazu ausgelegt, eine Ladung des Energiespeichers und Entladung des Energiespeichers an den Aktuator zu steuern,
- sie ist dazu ausgelegt, die Energiemenge für eine Ladung und Entladung zu steuern,
- sie ist mittels einer Programmiervorrichtung von extern konfigurierbar,
- sie weist eine Freigabeeinheit für die Entladung auf, und/oder
- die Entladung findet über mindestens zwei in Reihe geschaltete Impedanzen statt, wobei die Impedanzen mittels der Steuereinheit einstellbar sind.

5. Vorrichtung nach Anspruch 4, wobei die Steuereinheit die Entladung derart steuert, dass der Aktuator in einem Zeitraum von 0,1ms-5s elektromagnetische Wellen aussendet.

6. Vorrichtung nach mindestens einem der vorigen Ansprüche, wobei eine Entladung in mehr als einer Phase stattfindet.

7. Vorrichtung nach mindestens einem der vorigen Ansprüche, wobei der Aktuator mindestens eine der folgenden Eigenschaften aufweist:
- er umfasst mindestens eine Lichtquelle zur Aussendung der elektromagnetischen Wellen,
- er umfasst mindestens einen Strombegrenzer,
- er wird durch den Energiespeicher mit 1V bis zu 1500 V betrieben,
und/oder
- er ist von einem Gehäuse der Vorrichtung getrennt angeordnet und verfügt über einen Steckverbinder, wobei der Steckverbinder kompatibel ist zu einem Steckerausgang eines implantierbaren Geräts zur elektrischen Herzstimulation.

8. Vorrichtung nach Anspruch 7, wobei die Lichtquelle eine Reihenschaltung, oder eine Parallelschaltung, oder eine Kombination aus Reihenschaltung und Parallelschaltung von LEDs umfasst.

9. Vorrichtung nach Anspruch 7 oder 8, wobei die Lichtquelle eine Laserlichtquelle umfasst.

10. Vorrichtung nach mindestens einem der vorigen Ansprüche, wobei die Vorrichtung eine Telemetrieeinheit mit einem externen Gerät und/oder externen Datenzentrum umfasst.

11. Implantierbare Stimulationsvorrichtung zur Stimulation von Herzgewebe oder Nervengewebsstrukturen aufweisend Vorrichtung nach mindestens einem der vorigen Ansprüche.

12. Implantierbare Stimulationsvorrichtung nach Anspruch 11, wobei die Stimulationsvorrichtung über mindestens eine Stimulationselektrode aufweist.

13. Implantierbare Stimulationsvorrichtung nach Anspruch 11 oder 12, wobei die Stimulationsvorrichtung dazu ausgelegt ist, Stimulation von Herzgewebe hervorzurufen, wobei die Stimulation
- mittels des Aktuators durch elektromagnetische Wellen oder
- mittels elektrischer Stimulation,
wobei die Stimulation durch elektromagnetische Wellen und durch elektrische Stimulation einzeln, nacheinander oder zeitgleich stattfindet,
wobei die Stimulation in einem Areal oder mehreren Arealen des Gewebes stattfindet.

14. Implantierbare Stimulationsvorrichtung nach mindestens einem der Ansprüche 11 bis 13, weiter umfassend
- einen Steckkontakt kompatibel für einen Aktuator nach einem der Ansprüche 1 bis 7, und/oder
- einen Steckkontakt kompatibel für eine Elektrode für elektrische Stimulation, und/oder
- Aktuator und eine Elektrode.
